# EUROPEAN PATENT SPECIFICATION

(11) **EP 3 634 290 B1**
(45) Date of publication and mention of the grant of the patent: **24.01.2024**
(21) Application number: 18734727.3
(22) Date of filing: 07.06.2018
(51) Int. Cl.: A61B 18/20

(54) **SYSTEM FOR FAT REDUCTION AND IMPROVING SKIN LAXITY**
SYSTEM ZUR FETTVERMINDERUNG UND VERBESSERUNG DER HAUTSCHLAFFHEIT
SYSTÈME POUR RÉDUCTION DE L'ADIPOSITÉ ET AMÉLIORATION DU RELÂCHEMENT CUTANÉ

(30) Priority: 07.06.2017 US 201762516665 P
(43) Date of publication of application: 15.04.2020
(73) Proprietor: Cynosure, LLC, Westford, MA 01886 (US)
(72) Inventor: CHEN, Bo, Mountain View California 94041 (US); SIERRA, Rafael Armando, Gulfport Florida 33707 (US)
(74) Representative: Boult Wade Tennant LLP
(86) International application number: PCT/US2018/036516
(87) International publication number: WO 2018/226995

(56) References cited:
- WO-A1-2015/153620
- WO-A2-2014/149021
- US-A1- 2013 066 237
- US-A1- 2013 079 852

## Description

### FIELD

The present disclosure relates generally to systems and methods for applying energy (e.g., electromagnetic radiation such as laser radiation in the visible and near infrared wavelengths) to treat, for example, body areas having bulges and fat deposits and/or loose skin (skin laxity).

### BACKGROUND

The benefit of being able to raise and/or lower the temperature in a selected region of tissue for various therapeutic and cosmetic purposes has been known for some time. For instance, heated pads or plates or various forms of electromagnetic radiation, including microwave radiation, electricity, infrared radiation and ultrasound have previously been used for heating subdermal muscles, ligaments, bones and the like to, for example, increase blood flow, to otherwise promote the healing of various injuries and other damage, and for various therapeutic purposes, such as frostbite or hyperthermia treatment, treatment of poor blood circulation, physical therapy, stimulation of collagen, cellulite treatment, adrenergic stimulation, wound healing, psoriasis treatment, body reshaping, non-invasive wrinkle removal, etc. Heating may be applied over a small localized area, over a larger area, for example to the hands or feet, or over larger regions of tissue, including the entire body.

While optical and near infrared (NIR) radiation (collectively referred to hereinafter as "optical radiation") is generally both less expensive, and being non-mutagenic, safer than microwave radiation, the use of optical radiation has heretofore not been considered suitable for most applications involving heating of tissue at depth, the term "tissue at depth" as used herein meaning tissue at the border zone of the dermis and hypodermis, some of which tissue may be in the lower dermis, mostly at a depth deeper than 1 mm, and tissue below this border zone to a depth of up to about 50 mm.

In particular, optical radiation has not been considered suitable because such radiation is both highly scattered and highly absorbed in surface layers of tissue. As a result, these properties preclude significant portions of optical radiation from reaching the tissue regions at depth to cause heating thereof. In view of the energy losses due to scattering and absorption, substantial optical (including NIR) energy is applied in order for enough such energy to reach a region of tissues at depth to have a desired effect. However, precise modulation of the treatment temperatures in the treatment region (and regions surrounding the treatment region) has heretofore been difficult such that photothermal treatments in tissue regions at depth may not be fully efficacious and/or may cause undesirable damage to the tissue surrounding the treatment region. For these reasons, optical radiation has had limited value for therapeutic and cosmetic treatments on tissue at depth. In this context, reference is made to documents WO2015/153620 A1, WO2014/149021 A2 and US2013/0066237 A1.

The present disclosure addresses these technical problems and other challenges associated with the use of radiation in general and optical radiation in particular in the context of tissue heating applications targeting heating at various depths.

### SUMMARY

The invention is defined in the appended claims and relates to a non-invasive body contouring system. In accordance with various aspects of the present teachings, methods and systems are disclosed herein to target skin laxity and areas of relatively small bulges of fat tissue including fatty tissue that is relatively shallow relative to the skin surface (e.g., submental area, face, and neck). In accordance with various aspects of the present teachings, methods and systems are disclosed herein that have been discovered to be surprisingly effective in generating a desirable temperature profile in a target region (e.g., moderate hyperthermia in a range of about 42-47 °C) by providing a dynamic balance of heating (via the application of optical radiation to the skin surface) and cooling, while substantially confining treatment temperatures to the treatment region, e.g., about, adjacent to, or below the dermal-hypodermal junction (D/H junction).

As discussed otherwise herein, the present teachings, including thermal cycling, can be used to provide a targeted treatment region that reduces unwanted tissue damage outside the target region to provide particularly beneficial treatments that can simultaneously reduce fatty deposits (e.g., through lipolysis) and tighten the skin (e.g., through the increased production of collagen) while minimizing patient discomfort and unintended damage, for example, within the epidermis region above the targeted region and within the portion of the hypodermis below the targeted treatment region via the combination of one or more the following treatment parameters: total treatment time, duration of heating phases within the total treatment time (the cycle time of the heating phase and the cycle time of the non-heating and/or cooling phase), wavelength, and power of the applied optical radiation, and temperature of the skin surface cooling. In one aspect, the electromagnetic radiation is delivered and the cooling phase is controlled by a non-invasive body contouring system.

In order to enable photothermal treatment of tissue regions at depth (e.g., hyperthermic treatment of fatty tissue), various aspects of the present teachings provide methods and systems for modulating the application of radiation (or modulating the intensity of the radiation applied to the tissue) over an extended treatment time (e.g., in a range from about 20 minutes to about 30 minutes). By way of non-liming example, the photothermal treatment of hypodermal tissue (e.g., subcutaneous fatty tissue) and dermis can raise the mean tissue temperature at a treatment site at depth above about 40°C., e.g., from about 40°C to about 48°C, or from about 42°C to about 47°C by applying laser irradiation (e.g., having a central wavelength of about 1060 nm or about 1210 nm) to the treatment site to maintain this supraphysiological temperature (greater than 37°C) at the treatment site during the treatment duration.

In some aspects, for example, the treatment radiation can be applied over a relatively long duration, for example, up to and greater than 30 minutes though applicants have surprisingly discovered that in accordance with various aspects, a total treatment time of less than 30 minutes (e.g., from about 20 minutes to about 30 minutes, or from about 20 to about 25 minutes, or from about 25 to about 30 minutes) may be preferable to achieve the desired depth of treatment, for example, to trigger heat-induced injury in fatty tissue that causes the adipocytes to undergo apoptosis or lipolysis and/or to stimulating the hypodermal tissue about, adjacent to, or below the D/H junction for the production of collagen and hence skin thickening that can provide an appearance of tighter skin, while avoiding the formation of nodules. The residual cellular debris is gradually removed by the body through inflammation and the resultant immune system clearing process, which can take weeks to months depending on the patient and the extent of injury at the site. Since the regeneration process of adipose tissue is very slow (over years), the total volume of fat within the treatment area decreases due to loss of adipocytes that would otherwise act as storage units for fat.

Since the techniques described above involve applying treatment energy through the patient's skin surface, peak temperatures generally occur at or near the patient's skin surface, though due to thermal conduction, the extent of the thermal effect in tissue (e.g., up to about 3 cm) can be much deeper than optical penetration depth alone. As part of the development of the system and methods disclosed herein, particularly efficacious treatment parameters have been discovered. Specifically, the cooling and modulation of applied radiation to the skin surface can control the conduction of heat throughout the tissue layers by a time- and spatial-dependent process. For example, in various embodiments, such cooling and modulation of applied radiation confine and center the temperature gradient about the target tissue region at a temperature in a range from about 42-47 °C during the course of treatment. In contrast, adjacent, non-targeted, tissues above and/or below the target tissue region are substantially maintained below a treatment threshold.

Moreover, because the maximum tolerable temperature in tissue is limited by perceived patient discomfort, the treatment parameters have been discovered to create a temperature gradient where the peak temperature is located at the desired depth so as to help ensure that the targeted tissue achieves the maximum target temperature. Because it is desirable to confine the hyperthermic treatment to the target tissue while keeping temperatures of dermal tissue above the targeted tissue at depth below the treatment or injury threshold, the electromagnetic treatment parameters (such as radiation pattern, fluence, total exposure time, etc.) can be modulated over the extended treatment time, and in some aspects by taking into account the cooling rate on the skin surface, such that an optimal temperature profile/gradient in the target tissue (e.g., about, adjacent and/or below the D/H junction) can be achieved during the treatment.

As noted above, the optical radiation (e.g., laser light) utilized in accordance with the present teachings can exhibit a specific wavelength that is selectively or preferentially absorbed by the targeted tissue, such as the hypodermal tissue (e.g., subcutaneous fat tissue) about, adjacent and/or below the D/H junction, or adipocytes below the D/H junction, with less absorption and therefore less thermal effect on the surrounding tissues (such as epidermis). In various aspects, for example, the light source can generate radiation exhibiting a central wavelength at about 1210 nm (e.g., 1210 nm +/- 5 nm, 1210 nm +/- 10 nm, 1210 nm +/- 20 nm, or 1210 nm +/- 50 nm), which can be preferable due to its differential absorption properties in skin and fat that can generate a sharper temperature gradient at a target region at the D/H junction relative to other wavelengths, thereby substantially confining damage to the region of the D/H junction only with minimal collateral damage to adjacent tissue layers. For some deeper treatments that substantially confine the treatment temperature range (e.g., about 42 to about 47 C) within fat below the D/H junction but also generates a less sharp temperature gradient, the light source can generate radiation preferably exhibiting a central wavelength at about 1060 nm (e.g., 1060 nm +/- 5 nm, 1060 nm +/- 10 nm, 1060 nm +/- 20 nm, or 1060 nm +/- 50 nm) due to its differential absorption properties in skin and fat.

Alternatively, suitable systems can utilize a wavelength within the range of about 800 nm to about 1300 nm, or from about 800 nm to about 1150 nm, selected based on tissue penetrance, and exemplary power densities from about 0.5 to about 10 W/cm², or from about 0.5 to about 5 W/cm², or from about 1 to about 2 W/cm², and a particularly useful range is about 0.9 to about 1.4 W/cm². In some aspects, selection of wavelength alone may not itself be sufficient to create a large enough energy absorption differential between target and non-target tissues so as to achieve the desired temperature gradient due, for example, to conduction of thermal energy to the adjacent tissues and/or absorption by non-targeted tissue.

As such, in various aspects of the present teachings, confining optimal treatment effects to the target region can be achieved with the assistance of modulating the radiation pattern by selectively and cyclically applying radiation during the total treatment time and/or by applying cooling to the skin surface continuously or intermittently during the total treatment time so as to avoid damage to surrounding non-target tissues. Approaches that increase the energy absorption differential and control heating at the treatment site while lessening collateral damage of non-target tissues can in some aspects involve modulating the radiation exposure through pulsed applications of laser light.

For example, the cycle can have duration of less than about 20 seconds, less than about 15 seconds, or less than about 12 seconds, with the EMR being applied to the patient's skin for about 30%, about 40%, about 50%, about 60%, and about 70% of each cycle. To maintain an appropriate hyperthermic temperature range in the target tissue (e.g., about 42-47°C in the treatment region such as a fat layer or about the D/H junction) while avoiding pain and other unwanted side effects related to overheating, the laser can be modulated such that it can be pulsed so as to generate an on/off pattern or by modulating the intensity of the laser (e.g., between a high intensity and low intensity), which causes the temperature to cycle within the appropriate hyperthermic temperature range, as disclosed for example in U.S. Pub No. 20080103565 entitled "Method and Apparatus for Treatment of Cutaneous and Subcutaneous Conditions" and U.S. Pub. No. 20070213792 entitled "Treatment of Tissue Volume with Radiant Energy," the teachings of which may be used in the context of the invention. With the laser on (or at a desired relatively high intensity), the temperature can rise to the upper limits of the desired range.

Further, a periodic pause in radiation (or a lowering of the intensity) permits temperatures in the target site (and non-target site) to drop. Optionally, cooling (especially of the upper non-target tissue) can be further enhanced by using external devices (e.g., contact cooling) to maintain the skin surface at a temperature in a range from about 38°C to about 42°C, or at about 40°C for example, while laser radiation can resume (or its intensity be increased) before the target tissue temperature drops below the appropriate hyperthermic temperature range. In some embodiments, radiation is delivered through the contact cooled surface, which continuously cools the skin surface. Alternatively, contact cooling can be modulated via pulse on and off in concert with the delivery of radiation. The pulses can be repeated for the duration of the treatment.

In accordance with various aspects of the present teachings, the methods and systems can preferably utilize a near infrared laser having a central wavelength in a range from about 800 nm to about 1300 nm, or from about 800 nm to about 1150 nm, operated at a variety of effective power densities, e.g., depending on the radiation's on/off schedule, each cycle of which can last up to about 20 seconds, less than about 15 seconds, or less than about 12 seconds, with the EMR being applied to the patient's skin for about 30%, about 40%, about 50%, about 60%, and about 70% of each cycle. Additionally, a cooling mechanism maintained at a temperature in a range from about 20-35°C can be applied to the patient's skin during treatment (concurrently or between the applications of radiation during the on/off cycle) to maintain the skin surface at a temperature from about 38°C to about 42°C, or at about 40°C for a total treatment time in a range of from about 20 minutes to about 30 minutes (e.g., about 25-30 minutes).

For example, in accordance with various aspects of the present teachings, the methods and systems preferably utilize a near infrared laser having a central wavelength at about 1210 nm operated at a power density in a range of about 0.5-5 W/cm² (e.g., about 1-2 W/cm², or about 1-2.5 W/cm²), with the radiation being intermittently applied to the skin surface over duty cycles less than about 15 seconds in duration (e.g., 5 seconds on and 5 seconds off, 6 seconds on and 4 seconds off), while continuously applying a cooling mechanism maintained at a temperature in a range from about 20-30°C (e.g., about 20-25°C to maintain the skin surface at a temperature from about 38°C to about 42°C, or at about 40°) for a total treatment time in a range of from about 20 minutes to about 30 minutes (e.g., about 25-30 minutes).

In various preferred aspects of such a treatment, it has been surprisingly found that the treatment region damage is largely confined to region about, adjacent and/or below the D/H junction (e.g., the top of the treatment damage region begins directly adjacent the D/H junction at a depth that ranges from about 0 cm to about 0.5 cm from the skin surface and goes to a bottom of the treatment damage region that ends at a depth in the range from about 3 mm to about 1 cm, or from about 3 mm to about 8 mm) and results in an inflammation response that results in a substantially-increased deposition of collagen several weeks after the treatment relative to known previous treatments.

In accordance with other exemplary aspects of the present teachings, the methods and systems can generate a deeper treatment zone (e.g., maintaining tissue at a depth of about 1-3 cm from the skin surface at a temperature range from about 42 to about 47°C) utilizing a near infrared laser having a central wavelength at about 1060 nm operated at a power density in a range of about 0.5-5 W/cm² (e.g., about 1-2.5 W/cm²), with the radiation being intermittently applied to the skin surface over duty cycles less than about 15 seconds in duration (e.g., 5 seconds on and 5 seconds off), while continuously applying a cooling mechanism maintained at a temperature in a range from about 35°C to maintain the skin surface at a temperature from about 38°C to about 42°C, or about 40°C for a total treatment time in a range of from about 20 minutes to about 30 minutes (e.g., about 20-25 minutes). In various preferred aspects of such a treatment, it has been surprisingly found that damage is largely confined to hypodermal tissue (e.g., fat tissue) at a depth below the H/D junction and results in a significant reduction of fatty deposits relative to known previous treatments and also in formation of collagen.

With such extended treatment times, it may also be desirable that at least some, if not all, of the treatment can be accomplished hands-free and/or at times by the practitioner. By way of example, a hands free system in accordance with various aspects of the present teachings could enable the practitioner to start treatment of a first patient with a first system, and allow the practitioner to attend to or treat a second subject during the first subject's relatively long treatment time. In various aspects, such a substantially unattended approach can reduce the costs associated with treatment by freeing up the practitioner's time and potentially enable a less skilled practitioner to be able to conduct a majority of the treatment. For example, a less skilled practitioner can check in with and talk to the patient, to get a sense of the patient's comfort and then call in a more skilled practitioner to adjust the treatment parameters if necessary.

In accordance with some aspects of the present teachings, the systems and methods for relatively hands-free and/or substantially unattended treatment described herein can provide treatment that is reliable, safe, and/or relatively comfortable to the patient over the length of the treatment time. In addition, various aspects of the systems and methods disclosed enable customization so as to fit various body areas requiring treatment and/or the isolation of the target treatment area.

In accordance with various exemplary aspects of the present teachings, a system for substantially unattended treatment of body tissue is provided according to the methods described herein. In various aspects, the system comprises housing and at least one source of electromagnetic radiation for generating treatment energy contained within the housing. The system also comprises one or more applicators, each of which can be adapted to be placed in proximity to a treatment region of tissue of a patient's body and comprising an optical window having a skin-contacting surface through which the treatment energy is transmitted from the applicator to the treatment region.

In one aspect, a plurality of umbilical cords, each of which extends from the housing to a distal end coupled to one of the plurality of applicators, defines a conduit through which treatment energy generated by the at least one electromagnetic radiation source can be delivered from the housing to the applicator (e.g., through at least one optical waveguide extending through the conduit). In various aspects, the system can further comprise a cooling mechanism configured to cool the skin-contacting surface of the applicators when performing treatment.

By way of non-limiting example, a fluid pathway can extend through the conduit for circulating cooling fluid between the housing and the applicator via the umbilical cord. In various aspects, the cooling mechanism can be configured to maintain the skin-contacting surface of the applicator at a temperature in a range of from about 20°C to about 35°C (e.g., so as to maintain the skin surface at a temperature in a range from about 38°C to about 42°C, or about 40°C). In various aspects, the cooling mechanism can circulate cooling fluid from a heater/chiller disposed within the housing into thermal contact with the skin-contacting surface so as to maintain the skin-contacting surface at the surface at the desired temperature.

In various aspects, the system can also comprise a frame configured to be coupled to the patient's body in a fixed position relative to the treatment region and defining at least one aperture into which a surface of the treatment region can extend. The frame and at least one applicator can be coupled to one another in a variety of manners, but are generally removably coupled such that at least a portion of the skin-contacting surface of the optical window is disposed in contact with at least a portion of the surface of the treatment region extending into the aperture upon coupling the applicator with the frame. In some aspects, for example, the frame and the applicator can comprise complementary mating features for removably coupling the applicator to the frame. By way of example, the frame can comprise a snap-fit coupling mechanism for removably coupling the applicator to the frame. In various aspects, the system can additionally comprise an adjustable belt configured to be coupled to the frame for securing the frame to the patient's body.

In accordance with various exemplary aspects of the present teachings, a method for treating body tissue is provided. In some aspects, for example, the present teachings provide a method for stimulating collagen production and/or reducing fatty deposits in a target region at depth of a patient's skin, comprising: applying electromagnetic radiation to the skin surface for a duration sufficient to initially raise a temperature of a target region about, adjacent, and/or below the D/H junction to a treatment temperature in a range from about 42°C to about 47°C. Thereafter, the target region can be maintained within the treatment temperature range for a treatment duration from about 20 minutes to about 30 minutes (e.g., about 25 minutes), wherein the target region is maintained within the treatment temperature range by modulating the electromagnetic radiation applied to the skin surface so as to cyclically cool and heat the target region. The duration of the cooling phase is in a range from about 3 seconds to about 15 seconds, or in a range of from about 5 seconds to about 10 seconds and the duration of the heating phase is in a range from about 3 seconds to about 15 seconds, or in a range of from about 5 seconds to about 10 seconds. After the treatment duration, the application of electromagnetic radiation to the skin surface is terminated.

In various aspects, the target region can be at a depth in a range of about 1 mm to about 1 cm below the skin surface. Alternatively or additionally, in some aspects the target region can be at a depth in a range of about 1 cm to about 3 cm below the skin surface. Alternatively or additionally, in some aspects the target region can be at a depth in a range of about 2 cm to about 3 cm below the skin surface. Alternatively or additionally, in some aspects the target region can be at a depth in a range of about 1 cm to about 2 cm below the skin surface.

In some aspects, the electromagnetic radiation applied to the skin surface can be modulated by adjusting the power of the electromagnetic radiation applied to the skin surface between a first power for a cooling duration and a second power for a heating duration so as to cyclically cool and heat the target region. By way of example, the second power of the electromagnetic radiation is in a range between about 1 W/cm² and about 5 W/cm², in a range between about 1 W/cm² and about 2.5 W/cm², in a range between about 1 W/cm2 and about 2 W/cm², and additionally, the power of the electromagnetic radiation can be substantially zero when the device is shut off.

In various aspects, the method can further comprise contacting a cooling surface through which the electromagnetic radiation is applied to the skin onto the surface of the patient's skin during the step of maintaining the target region within the treatment temperature range, wherein the temperature of the cooling surface can be maintained in a range of about 20°C to about 35°C. In some aspects, the temperature of the cooling surface is maintained in a range of about 20°C to about 25°C. In some aspects, the temperature of the cooling surface is maintained in a range of about 25°C to about 30 °C. Alternatively, the temperature of the cooling surface can be maintained at a temperature of about 35°C.

The cooling phase and applying energy phase can exhibit a variety of duty cycles. For example, the duration of the cooling phase can be in a range from about 3 seconds to about 10 seconds and the duration of the heating phase can be in a range from about 3 seconds to about 10 seconds. Further, in some aspects, the duration of the cooling phase can be in a range from about 3 seconds to about 7 seconds and the duration of the heating phase can be in a range from about 3 seconds to about 7 seconds. In some aspects, the duration of the cooling phase can be in a range from about 3 seconds to about 6 seconds and the duration of the heating phase is in a range from about 3 seconds to about 6 seconds.

For example, the duration of the cooling phase can be about 5 seconds and the duration of the heating phase can be about 5 seconds. Alternatively, in some aspects, the duration of the cooling phase can be about 4 seconds and the duration of the heating phase can be about 6 seconds. In some aspects, the cyclic portion of the heating/cooling phase can be preceded by an initial heating stage to initially raise the temperature of the target region to the treatment temperature. In some aspects, for example, the initial heating phase can exhibit a duration less than about 3 minutes (e.g., in a range of about 30 seconds to about 90 seconds, or in a range of about 20 seconds to about 40 seconds).

The electromagnetic radiation exhibits at least one wavelength in the near infrared range, for example, in a range from about 800 nm to about 1300 nm, from about 800 nm to about 1150 nm. By way of example, in various preferred aspects, the electromagnetic radiation can exhibits a wavelength of about 1210 nm (e.g., 1210 nm +/- 5 nm, 1210 nm +/- 10 nm, 1210 nm +/- 20 nm, or 1210 nm +/- 50 nm). Alternatively, in some aspects, the electromagnetic radiation can exhibit a wavelength selected from about 800 nm, about 940 nm, and about 1060 nm (e.g., 1060 nm +/- 5 nm, 1060 nm +/- 10 nm, 1060 nm +/- 20 nm, or 1060 nm +/- 50 nm).

In some exemplary aspects, the method can further comprise coupling a frame to a patient's body in a fixed position relative to a treatment region of tissue, the frame defining at least one aperture into which a surface of the treatment region extends. At least one applicator can be coupled to the frame, each applicator comprising an optical window having a skin-contacting surface through which treatment energy is configured to be transmitted from the applicator to the treatment region, wherein at least a portion of the skin-contacting surface of the optical window is disposed in contact with at least a portion of the surface of the treatment region extending into said aperture upon coupling with the frame. Thereafter, treatment energy can be transmitted to the portion of the surface of the treatment region extending through the aperture of the frame and disposed in contact with the skin-contacting surface of the optical window, the treatment energy being generated by at least one source of electromagnetic radiation disposed in a housing and delivered to the applicator via an umbilical cord extending from the housing to a distal end of the umbilical cord that is coupled to the applicator.

In some aspects, coupling at least one applicator to the frame can comprise coupling a plurality of applicators to the frame, wherein each of the applicators is associated with a different umbilical cord and a different aperture of the frame configured to isolate a different surface of the treatment region.

In various aspects, the housing can additionally comprise at least one arm extending from the housing for supporting the umbilical cords, the method further comprising disposing the arm above the patient's body when performing treatment. In some exemplary aspects, the arm can also comprise at least one brake in contact with each of the plurality of umbilical cords so as to maintain a desired amount of lead of each umbilical cord between the at least one brake and the applicator associated with each umbilical cord.

In some exemplary aspects, coupling the frame to the patient's body can comprise securing a belt coupled to the frame around at least a portion of the patient's body. By way of example, when the treatment region for tissue tightening and/or superficial fat treatment comprises one of submental, jowl, and neck tissue, the belt can be secured about the patient's head and/or neck. Alternatively, when the treatment region for tissue tightening and/or superficial fat treatment comprises abdominal tissue, the flanks, the under-bra area (in the back or in the front), the belt can be secured about the patient's torso. Finally, when the treatment region for tissue tightening and/or superficial fat treatment comprises tissue of the patient's arm (e.g., the under portion of the arm above the elbow) or leg (e.g., the knee, where the thighs meet and/or the saddle bag area), for example, the belt can be secured around the patient's arm or leg, respectively.

Additionally, the orange peel and/or mattress cover appearance associated with some forms of cellulite may be improved via the treatment regimes for tissue tightening and/or superficial fat treatment discussed herein. In various related aspects, the frame can comprise a hinge disposed between adjacent apertures, wherein coupling the frame to the patient's body further comprises adjusting the orientation of the apertures relative to each other (e.g., as the belt is tightened about the patient).

In various aspects, the method can also include coupling the frame to at least one mask configured to occlude a portion of the frame's aperture so as to prevent a portion of the surface of the patient's body from extending into the aperture and into contact with the optical window of the applicator. The unmasked portion of the mask can have an area smaller than each of the optical window of the applicator and the aperture of the frame associated with the mask, the method further comprising adjusting at least one of the size and shape of the unmasked portion (e.g., so as to customize the tissue to which the treatment energy is applied).

Although, the disclosure relates to different aspects and embodiments and other features as recited and depicted herein, it is understood that the each of the foregoing disclosed herein can be integrated together as a whole or in part, as appropriate. Thus, each embodiment disclosed herein can be incorporated in each of the aspects to varying degrees as appropriate for a given implementation.

These and other features of the applicant's teachings are set forth herein.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The skilled person in the art will understand that the drawings, described below, are for illustration purposes only. The drawings are not intended to limit the scope of the applicant's teachings in any way.
FIG. 1 depicts a plot of absorption coefficients versus wavelength for lipid and water in the NIR range.
FIG. 2A schematically depicts layers of the human skin.
FIG. 2B schematically depicts an exemplary treatment targeting a treatment region in accordance with various aspects of the present teachings.
FIG. 2C schematically depicts another exemplary treatment targeting a treatment region in accordance with various aspects of the present teachings.
FIG. 3 depicts an exemplary plot of tissue temperature for the target treatment during an exemplary treatment in accordance with various aspects of the present teachings.
FIG. 4A schematically depicts a typical treatment regime of a known, commercially-available device marketed under tradename SculpSure by Cynosure, Inc.
FIG. 4B schematically depicts an exemplary treatment targeting a treatment region in accordance with various aspects of the present teachings.
FIG. 4C schematically depicts an exemplary treatment targeting a treatment region in accordance with various aspects of the present teachings.
FIG. 5A is an ultrasound (US) image of an abdominal area one week after it was treated with the discussed with respect to FIG. 4A.
FIG. 5B is a US image of an abdominal area one week after it was treated in accordance with the exemplary treatment of FIG. 4B.
FIG. 5C is a US image of an abdominal area one week after it was treated in accordance with the exemplary treatment of FIG. 4C.
FIG. 5D is a US image showing the results of a separate Device 1 treatment study.
FIG. 6A is a US scan of untreated tissue.
FIG. 6B is a US scan the tissue of FIG. 6A immediately after the treatment of FIG. 5C.
FIG. 6C is a US scan the tissue of FIG. 6A six weeks after the treatment of FIG. 5C.
FIG. 6D is a US scan the tissue of FIG. 6A twelve weeks after the treatment of FIG. 5C.
FIG. 7A depicts the histology of a sample twelve weeks after the treatment of FIG. 4C.
FIG. 7B depicts the histology of another sample twelve weeks after the treatment of FIG. 4C.
FIG. 7C depicts the histology of an untreated abdominal tissue sample.
FIG. 7D depicts the histology of a sample near the untreated sample in FIG. 7C twelve weeks after the treatment of FIG. 4C.
FIG. 7E depicts the histology of another sample near the untreated sample in FIG. 7C twelve weeks after the treatment of FIG. 4C.
FIG. 7F depicts the histology of a sample twelve weeks after the treatment of FIG. 4B.
FIG. 7G depicts the histology of a sample twelve weeks after the treatment of FIG. 4B.
FIG. 8 depicts a spatial temperature distribution at various depths in a subject's abdominal tissue during an exemplary treatment.
FIG. 9 depicts a spatial temperature distribution at various depths in a subject's abdominal tissue during another exemplary treatment.
FIGS. 10A-10D represent US images of a single patient at various times following an exemplary treatment.
FIGS. 11A-11B represent MRI images of a single patient at various times following an exemplary treatment.
FIGS. 12A-B photographs of a patient prior to an exemplary treatment, and twelve weeks after the treatment.
FIGS. 13A-B photographs of a patient prior to an exemplary treatment, and twelve weeks after the treatment.
FIGS. 14A-B photographs of a patient prior to an exemplary treatment, and twelve weeks after the treatment.
FIG. 15 depicts an exemplary system for the non-invasive (or less-invasive) photothermal treatment for skin tightening and fat reduction in accordance with various aspects of the present teachings.
FIG. 16 depicts a schematic cross-section of a portion of the system of FIG. 15.
FIG. 17 depicts an exemplary applicator suitable for use with the system of FIG. 15

### DETAILED DESCRIPTION

It will be appreciated that for clarity, the following discussion will explicate various aspects of embodiments of the applicant's teachings, while omitting certain specific details wherever convenient or appropriate to do so. For example, discussion of like or analogous features in alternative embodiments may be somewhat abbreviated. Well-known ideas or concepts may also for brevity not be discussed in any great detail. The skilled person will recognize that some embodiments of the applicant's teachings may not require certain of the specifically described details in every implementation, which are set forth herein only to provide a thorough understanding of the embodiments. Similarly it will be apparent that the described embodiments may be susceptible to alteration or variation according to common general knowledge without departing from the scope of the disclosure. The following detailed description of embodiments is not to be regarded as limiting the scope of the applicant's teachings in any manner.

The term "about" and "substantially identical" as used herein, refers to variations in a numerical quantity that can occur, for example, through measuring or handling procedures in the real world; through inadvertent error in these procedures; through differences/faults in the manufacture of electrical elements; through electrical losses; as well as variations that would be recognized by one skilled in the art as being equivalent so long as such variations do not encompass known values practiced by the prior art. Typically, the term "about" means greater or lesser than the value or range of values stated by 1/10 of the stated value, e.g., ±10%. For instance, applying a voltage of about +3V DC to an element can mean a voltage between +2.7V DC and +3.3V DC. Likewise, wherein values are said to be "substantially identical," the values may differ by up to 5%.

In accordance with various aspects of the present teachings, systems and methods for providing photothermal treatment of tissue at depth are provided herein. In various aspects, systems and methods disclosed herein have been discovered to be surprisingly effective in generating a desirable temperature profile in a target region (e.g., moderate hyperthermia in a range of about 42-47°C) by providing a dynamic balance of heating (via the application of optical radiation to the skin surface) and cooling, while nonetheless substantially confining treatment temperatures to the treatment region.

Particularly beneficial treatments can simultaneously reduce fatty deposits (e.g., through lipolysis) and tighten the skin (e.g., through the increased production of collagen), while minimizing patient discomfort and unintended damage, for example, within the epidermis and hypodermis regions adjacent the treatment region via the combination of one or more the following treatment parameters: total treatment time, duration of heating phases within the total treatment time, wavelength and power of the applied optical radiation, cycling of heating and cooling phases, and temperature of the skin surface cooling.

In various aspects, the optical radiation (e.g., laser light) utilized in accordance with the present teachings can exhibit a specific wavelength that is selectively or preferentially absorbed by the targeted tissue (such as tissue about, adjacent and/or below the D/H junction or adipocytes below the D/H junction), with less absorption and therefore less thermal effect on the surrounding tissues (such as epidermis). In accordance with the present teachings, various other treatment parameters can also be utilized, for example, in conjunction with the selected wavelength to provide treatments resulting in drastic improvements in fat reduction and/or skin laxity for various areas of the body relative to known devices and methods (e.g., in the abdomen and in the submental area).

In certain aspects, for example, it has been found that a skin-contact surface of the radiation applicator can be maintained at a higher temperature than previously believed to provide sufficient cooling of the skin to maintain the skin surface temperature at a range of about 38°C to about42 °C, or at about 40°C during the treatment duration, while nonetheless maintaining the ability of the treatment region at depth to be maintained at a temperature in a range of about 42°C to about 47°C.

By way of example, skin-contacting surface applicator cooling mechanisms that can achieve the targeted skin surface temperature range of about 38 to about 42 °C, or at about 40°C during the treatment include a skin-contacting surface of the applicator in a range of 20-30°C, or in the range of 20-25°C during treatment with radiation of a wavelength of about 1210 nm and at about 30-35°C, or about 35°C during treatment with radiation of a wavelength of about 1060 nm. The different skin contacting surface temperature ranges are necessitated by the differing selectivity and differing absorption of the two wavelengths, 1210 nm and 1060 nm.

As described herein, additional parameters including total treatment time (e.g., about 25-30 minutes) can additionally be utilized to provide particularly effective results. By way of example, it has been discovered that a total treatment time of about 25-30 minutes can produce substantially improved cosmetic results in fat reduction and/or skin laxity relative to shorter treatment times, while extending the treatment time beyond about 30 minutes can result in nodule formation, which is undesirable. In various aspects of the present teachings, treatment parameters: total treatment time, duration of heating phases within the total treatment time, wavelength and power of the applied optical radiation, and temperature of the skin surface cooling. In various embodiments, the delivery of optical radiation, such as radiation from one or more laser sources, and the application and/or control of cooling phases are performed using a non-invasive body contouring system.

Since the techniques described herein involve applying treatment energy through the patient's skin surface, peak temperatures generally occur at or near the patient's skin surface, though due to thermal conduction, the extent of the thermal effect in tissue (e.g., up to about 3 cm) can be much deeper than optical penetration depth alone. Key to the present teachings is the discovery of particularly efficacious treatment parameters that the cooling and modulation of the application of radiation to the skin surface can control the conduction of heat throughout the tissue layers by a time- and spatial-dependent process that confine and center the temperature gradient about the target tissue region at a temperature in a range from about 42-47°C during the course of treatment, while adjacent tissues are substantially maintained below a treatment threshold. Moreover, because the maximum tolerable temperature in tissue is limited by perceived patient discomfort, the treatment parameters have been discovered to create a temperature gradient where the peak temperature is located at the desired depth so as to help ensure that the targeted tissue achieves the maximum target temperature.

Because it is desirable to confine the hyperthermic treatment to the target tissue while keeping temperatures of dermal tissue above the targeted tissue at depth below the treatment or injury threshold, the electromagnetic treatment parameters (such as radiation pattern, fluence, total exposure time, etc.) can be modulated over the extended treatment time, and in some aspects by taking into account the cooling rate on the skin surface, such that an optimal temperature profile/gradient in the target tissue (e.g., about, adjacent and/or below the D/H junction) can be achieved during the treatment.

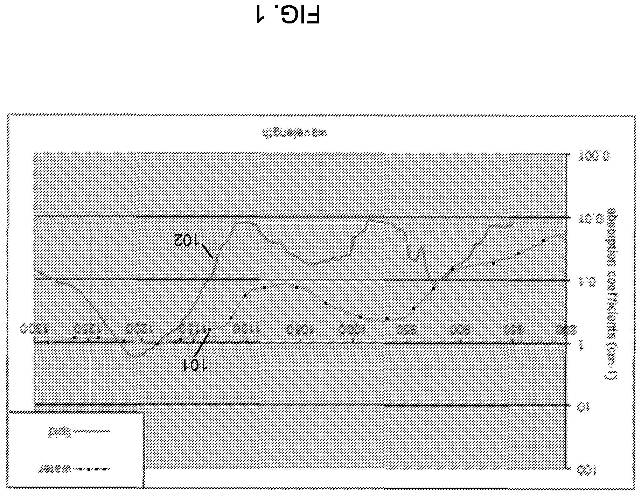

As noted above, optical radiation (e.g., laser light) utilized in accordance with the present teachings can exhibit a specific wavelength that is selectively or preferentially absorbed by the targeted tissue (such as hypodermal tissue about, adjacent and/or below D/H junction or adipocytes below the D/H junction), with less absorption and therefore less thermal effect on the surrounding tissues (such as epidermis). With reference now to FIG. 1, the absorption of NIR (e.g., having a wavelength about 800 nm to about 1300 nm) by water 101 and lipid 102 is depicted. Specifically, FIG. 1 shows the absorption curve of water and lipid at 800 nm-1300 nm.

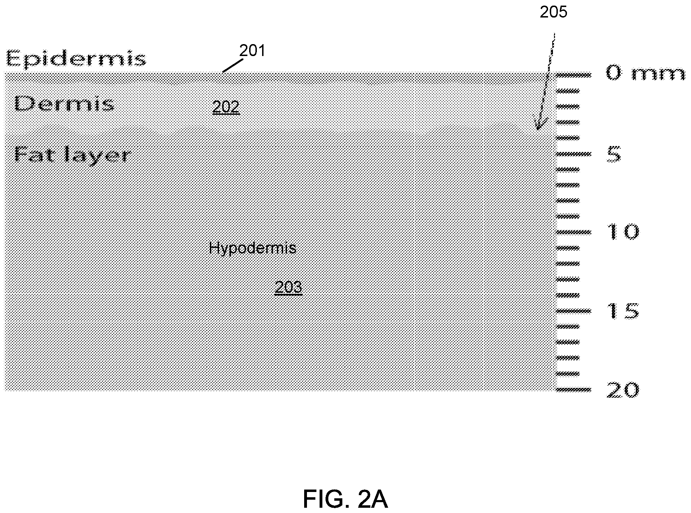

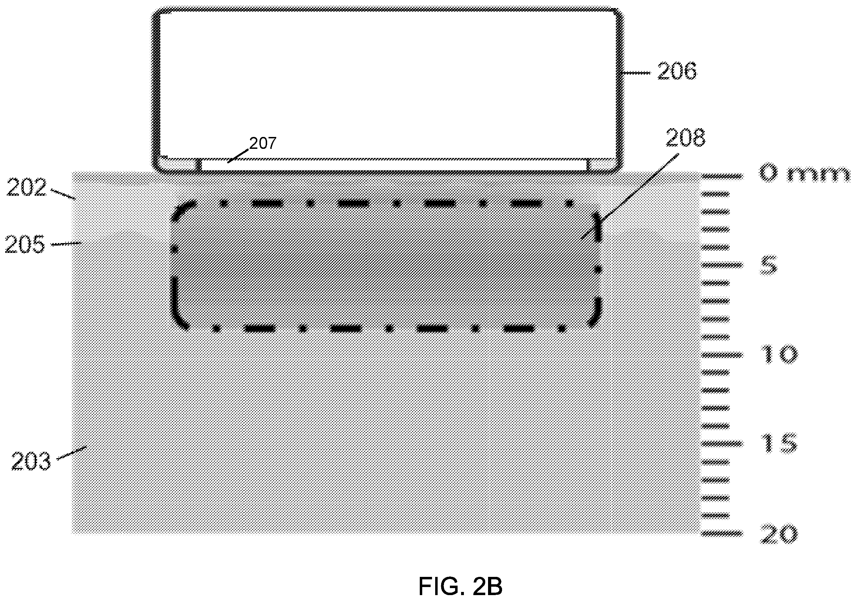

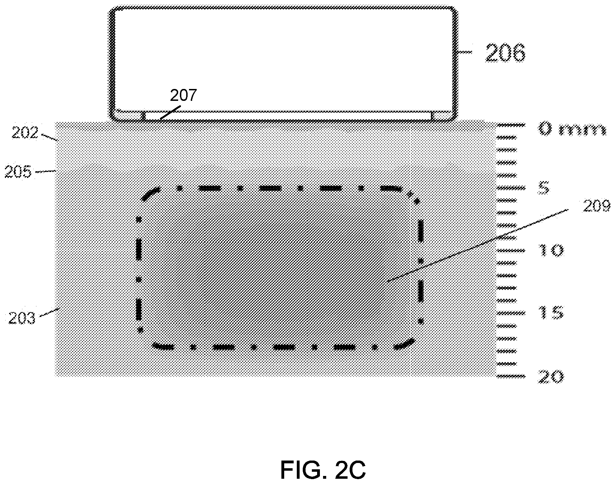

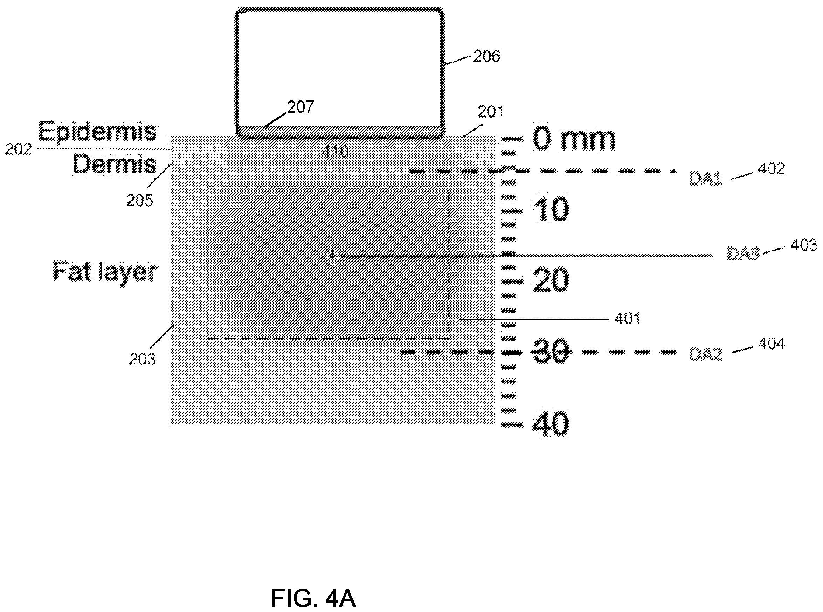

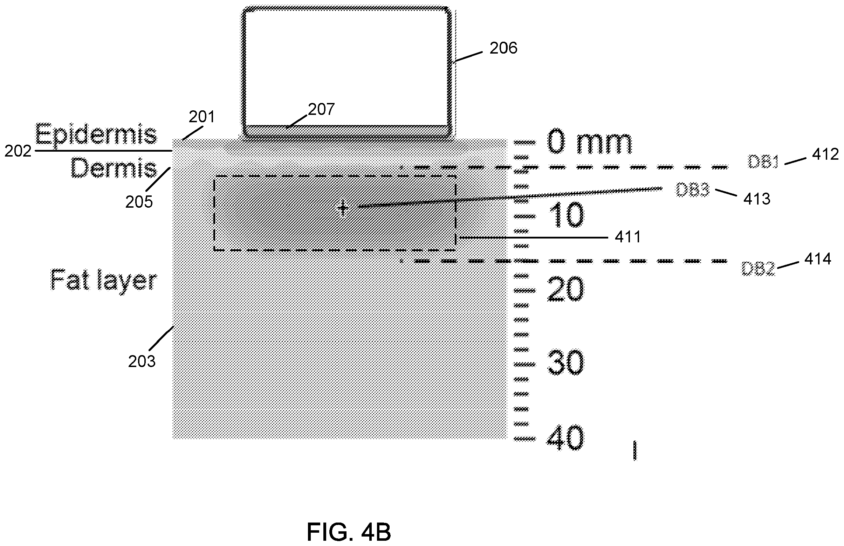

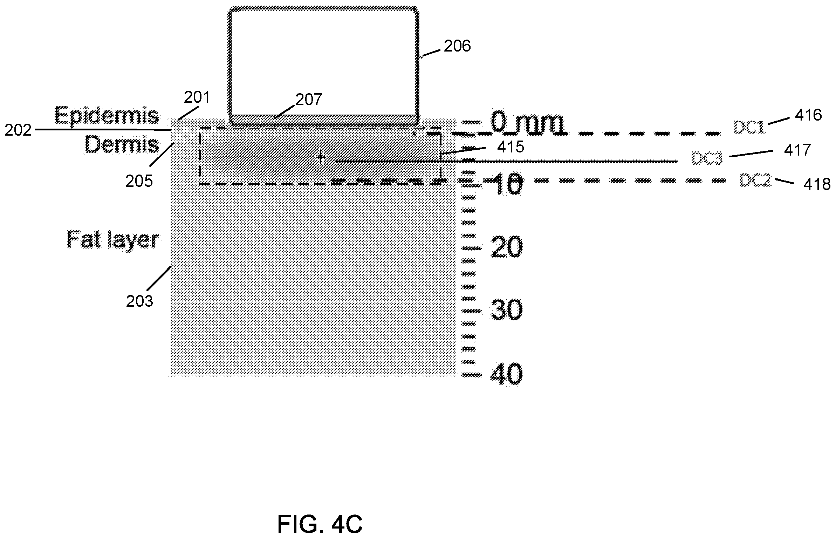

With reference now to FIG. 2A, various layers of human skin are depicted schematically. The uppermost layer 201 (i.e., near the skin surface) represents the epidermis, which typically extends below the skin surface to a depth in a range of about 0.5 mm to about 1.5 mm. The dermis 202 represents the next layer at depth and extends from the epidermis to a depth about 3-5 mm below the skin surface. The dermis is shown with a pink color in FIGS. 2A, 2B, 2C, 4A, 4B, and 4C. The dermis largely consists of connective tissue (e.g., collagen, elastin), but also includes capillaries, nerve endings, and hair follicles. Collagen is the most common structural component in the dermis and provides the skin with strength and flexibility, while elastin provides the skin with elasticity. Skin laxity is largely due to the loss or breakdown of collagen and elastin in the dermis, and can result from various intrinsic factors (e.g., age) and extrinsic factors (e.g., UV exposure). The hypodermis 203, which is the thickest part of the skin and largely contains fat tissue, is adjacent to the dermis and meets the dermis at what is referred to as the dermal/hypodermal junction (D/H junction) 205 and the hypodermis extends from the dermis to a depth up to about 50 mm. The hypodermis 203 is shown with an orange / brownish color in FIGS. 2A, 2B, 2C, 4A, 4B, and 4C.

With reference now to FIG. 2B, in various aspects of the present teachings, the target region 208 can be disposed about and/or adjacent the D/H junction 205 (e.g., from a top portion of the treatment region is adjacent the D/H junction 205 to a depth (measured from the surface of the skin) in a range from about 3 mm to about 1 cm, or from about 3 mm to about 8 mm. In accordance with various aspects of the present teachings, an applicator 206, such as for example a handpiece, is placed in contact with the skin surface can be configured to apply optical radiation (and in some aspect surface cooling) such that the treatment regime generates a temperature distribution in the patient's skin that maintains the target tissue 208 at a temperature in the range of about 42-47°C for the treatment duration (e.g., about 20-30 minutes, or about 25 minutes). The target region 208 is shown with a reddish color in the figures.

This target tissue 208 is bounded by the dotted lines as shown and, after receiving optical radiation, becomes a thermally affected treatment volume. In such aspects, the resulting post-treatment inflammatory response can, for example, stimulate the production of collagen in the dermis 202 and/or hypodermis 203 (i.e., the subcutaneous fat), which can lead to the appearance of dermal thickening that reduces the appearance of skin laxity. The applicator 206 can include one or more cooling devices or cooling elements 207. The cooling element results in tissue cooling as shown by blue region 410. The cooling elements are controlled to be at a lower temperature relative to that of the treatment temperatures achieved. Skin cooling is achieved by heat transfer from the skin to the cooling surface of the applicator.

In some particular aspects, such a treatment regime can also be effective to simultaneously heat fat cells within the hypodermis adjacent to or below the D/H junction to stimulate lipolysis, which can lead to the destruction of fat cells. While decreasing fatty deposits within subcutaneous tissue can sometimes increase the appearance of loose skin (e.g., around the jowls following significant weight loss or due to treatments described herein which reduce the fatty deposits that cause the appearance of a double chin), it has been discovered that a treatment regime targeting tissue about or adjacent the D/H junction 205 can simultaneously increase the dermal thickness and/or the thickness of the treated hypodermis (i.e., the subcutaneous fat), because the new collagen created about, or adjacent to the D/H junction 205 will contribute to skin thickness so as to tighten the skin despite the loss of fatty tissue, thereby providing a particularly beneficial cosmetic result. As will be appreciated from FIG. 2B, such a treatment regime can create a sharp temperature gradient in the tissue such that overlying tissue in the epidermis and underlying tissue in the hypodermis are not exposed to the hyperthermic treatment temperatures.

In various alternative aspects of the present teachings, with reference now to FIG. 2C, the treatment regime can be configured to target deeper regions 209 below the D/H junction 205 (e.g., a top portion of the treatment region is at a distance below the D/H junction 205 to a depth of about 1 cm to about 3 cm from the skin surface) to maintain this region at hyperthermic temperatures for the treatment duration (e.g., about 20-30 minutes, or about 25 minutes), which can lead to destruction of fatty cells within the hypodermis (e.g., via lipolysis). As shown in FIG. 2C, such a treatment regime can create a temperature gradient (though not as narrowly tailored and/or confined as the temperature gradient shown in FIG. 2B) in the tissue such that overlying tissues in the dermis and epidermis are not exposed to the hyperthermic treatment temperatures.

In light of the differential absorption by fat 102 and water 101 of the various wavelengths of near infrared (NIR) shown above in FIG. 1, it will be appreciated that the selection of particular wavelengths can generally be utilized to target tissues at different depths. In some aspects, for example, the light source can generate radiation exhibiting a central wavelength at about 1210 nm, which can be utilized to generate the shallower, sharper temperature gradient adjacent the D/H junction as shown in FIG. 2B, for example. A central wavelength at about 1210 nm is suitable, due to its increased absorption in fat relative to water, as can be seen in the absorption curve in FIG. 1. For example, due to the selectivity of 1210 nm to fat tissue, energy is preferentially deposited in the region of the hypodermis (hypodermis is subcutaneous fat tissue) that lies directly adjacent the D/H junction 205.

It has been discovered that such a sharp temperature gradient can be effective to confine the damage about or adjacent to the D/H junction 205 with only minimal collateral damage to adjacent tissue layers. Because a treatment in the hypodermis (e.g., subcutaneous fat) that is directly adjacent to the D/H junction 205 is close to the dermis and collagen is the most common structural component in the dermis, the treatment adjacent the D/H junction 205 enjoys an excellent source of collagen cells from the adjacent dermis and this is expected to improve the quantity of collagen generation relative to a treatment below the D/H junction 205 as shown in FIG. 2C.

For deeper treatments that substantially confine the treatment temperature range (e.g., about 42°C to about 47°C) within fat below the D/H junction 205 but that generate a less sharp temperature gradient, the light source can generate radiation preferably exhibiting a central wavelength at about 1060 nm due to the relatively decreased absorption of this wavelength in the upper layers of tissue which contain relatively more water. Other wavelengths that could provide a similarly less sharp temperature gradient, because they have a relatively decreased absorption in the upper layers of tissue which contain relatively more water are wavelengths within the range of from about 800 nm to about 1150 nm.

As noted above and discussed otherwise herein, applicants have discovered the criticality of particularly efficacious treatment parameters (e.g., wavelength, treatment duration, treatment pattern, laser power, selective cooling) that is effective to control the conduction of heat throughout the tissue layers by time- and spatially-dependent processes. In such a manner, the present teachings can enable effective to confine and center the temperature gradient about the target tissue region at a temperature in a range from about 42-47°C during the course of treatment, while adjacent tissues are nonetheless substantially maintained below a treatment threshold.

Such a desired temperature profile in a region at depth can be generated due to absorption of the treatment radiation by the target region and the resulting thermal conduction therefrom (which can result in a thermal treatment effect in tissue deeper than optical penetration depth alone). In various aspects, the dynamic balance of heating (e.g., by controlling power and or pulse pattern) and cooling (e.g., by controlling cooling temperature) can provide a particularly effective treatment regime that establishes a spatial temperature gradient such that the peak temperature is located at the depth of the target tissue.

The setting of cooling, laser power, and total treatment time can be provided to establish and maintain the desired temperature distribution within the tissue. In various preferred aspects, the peak temperature occurs about, adjacent to, and/or below the D/H junction 205 and has been discovered to be particularly effective at simultaneously improving the appearance of skin laxity and/or reducing fat deposits.

In certain exemplary aspects, selection of treatment time has been shown to be critical in providing some of the particularly beneficial effects of methods and systems in accordance with the present teachings. In particular, the treatment radiation can be applied over a relatively long duration, for example, up to and greater than 30 minutes though applicants have surprisingly discovered that in accordance with various aspects, a total treatment time of less than 30 minutes (e.g., from about 20 minutes to about 30 minutes, or from about 20 to about 25 minutes, or from about 25 to about 30 minutes) may be preferable to achieve the desired depth of treatment and to trigger heat-induced injury that causes the adipocytes to undergo apoptosis or lipolysis and/or that stimulates the production of collagen. In turn, collagen production promotes skin thickening that can provide an appearance of tighter skin, while avoiding the formation of nodules.

Creating sufficient damage adjacent to the D/H junction 205 that can lead to a post-treatment inflammatory response is desirable for various applications. Such controlled damage can be achieved by utilizing a treatment time greater than about 20 minutes, while reducing deleterious effects that were discovered to occur after about 30 minutes of the target tissue being maintained in the hyperthermic treatment temperature range (e.g., 42-47°C). For example, treatment times greater than about 30 minutes can produce undesirable damage to tissues adjacent to the treatment region.

In experiments in accordance with various aspects of the present teachings, the applicants' studies showed inflammation can be created before other structures (e.g., blood vessels, nerve, etc.) were damaged by the treatment regime. Studies also showed that hyperthermia treatment much longer than about 30 minutes caused the development of palpable nodules in the hypodermis layer consistent with clinical findings of fat necrosis. These nodules were observed at 1 month post-treatment and did not resolve at 6 months. The 6-month nodule, resulting from higher dosage exposure (treatment time 45 minutes), demonstrated extreme pathological changes with "ghost-like" mummified fat cells at the center, surrounded by fibrosis and cystic spaces consistent with encapsulated fat necrosis.

For treatment time less than about 20 minutes, histological analysis and gross measurement of the treatment zone demonstrated a small area of effect or no discernible inflammation occurring post-treatment. Thus, treatment time less than about 20 minutes, or less than about 17 minutes, or less than about 15 minutes is believed to provide ineffective results. In particularly preferred aspects, a treatment time of 20-25 minutes can be utilized to generate inflammation without long term undesirable side effects. Accordingly, about 20 to about 25 minutes is a target range for a given session that includes one or more cooling phases.

In addition to total treatment time, applicants have found that modulating laser exposure (e.g., pausing application of radiation periodically during an on/off cycle or decreasing its intensity during a high/low cycle) throughout the treatment helps increase or maximize patient comfort and to effectively control thermal conduction from the tissue. In accordance with the present teaching, cycles of applying (and removing radiation or lessening radiation) takes the following consideration into account. First, the off time (or reduced intensity time) needs to be short enough as compared to thermal relaxation time so as to maintain the temperature in the treatment range (e.g., 42-47°C). Thermal relaxation time can generally be considered to be the time required for the temperature of a tissue structure to decay by a given amount, 50% of which is commonly a result of conduction. The thermal relaxation time is therefore a convenient parameter that can be used to characterize the length of the time heat is primarily confined to the target tissue.

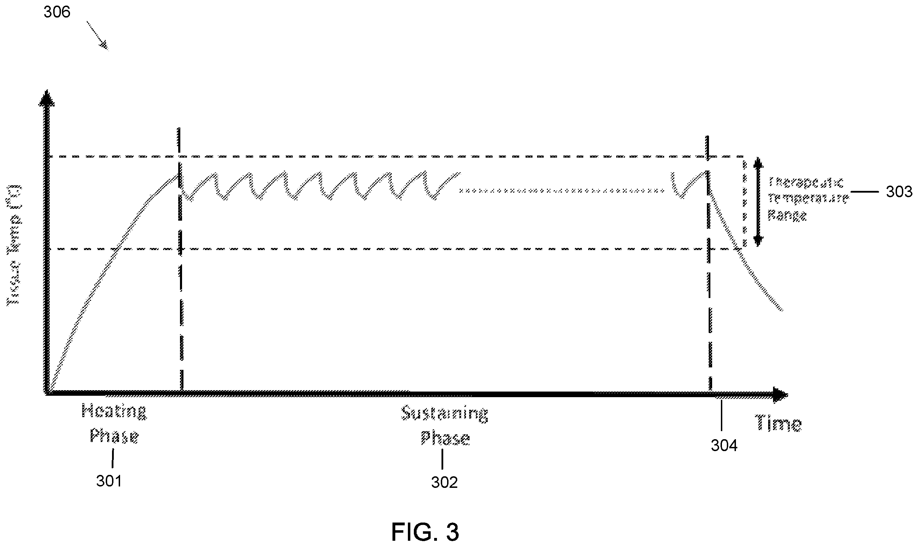

Secondly, the off time needs to be long enough to provide pain relief to a subject feeling increased thermal sensation at the end of the laser-on period. With reference now to FIG. 3, an exemplary plot 306 of tissue temperature by modulation methods in accordance with various aspects of the present teachings is depicted. As shown, the target tissue can be raised to the treatment temperature range (e.g., 42-47°C) during an initial heating or build phase 301. Depending on laser power, for example, in the initial heating or build phase 301 the treatment radiation can be applied continuously (e.g., for a period less than three minutes, for a period less than two minutes, in a range of about 30 seconds to about 90 seconds, or in a range of about 20 seconds to about 40 seconds).

After tissue temperature is brought up to treatment range 303, the dynamic heating and cooling phase can be applied to maintain the target tissue in the desired treatment temperature range 302. During the laser on time in each cycle, the tissue temperature can increase to a peak temperature before the off time starts. Prior to the tissue temperature dropping outside of the treatment range 304, laser radiation can again be applied to the skin (e.g., the laser can be turned on) so that the tissue temperature stays in treatment range during the remainder of the treatment duration (i.e., the sustain phase).

The laser can be modulated to be turned on and off at various duty cycles for various cycle durations. For example, for relatively shallower treatments (e.g., in which the target region is about, adjacent to or a small distance below the D/H junction 205), it can be preferable to have the cycle time be less than about 15 seconds (e.g., about 10 seconds), with the EMR being applied to the patient's skin for about 30%, about 40%, about 50%, about 60%, and about 70% of each cycle. For example, the duration of each of the heating and cooling phases (e.g., the on-off cycle) can be in a range from about 3 seconds to about 10 seconds, in a range from about 3 seconds to about 7 seconds and the duration of the heating phase can be in a range from about 3 seconds to about 7 seconds, or in a range from about 3 seconds to about 6 seconds. In some aspects, the duration of the heating phase can be about 5 seconds and the duration of the cooling phase can be about 5 seconds. Although reference is made to a laser herein, other sources of optical radiation can be used in various embodiments.

Alternatively, in some aspects, the duration of the heating phase can be about 6 seconds and the duration of the cooling phase can be about 4 seconds. Moreover, as noted above, the cyclic portion of the heating/cooling phase can be preceded in some treatment regimes by an initial heating stage (e.g., having a duration in a range or about 3 minutes, or in a range of about 2 minutes, or in a range of from about 30 seconds to about 90 seconds, or from about 20 seconds to about 40 seconds) to initially raise the temperature of the target region to the treatment temperature.

As noted above, the method can further comprise cooling the skin surface to further enhance the control of the temperature gradient within the tissue, especially with respect to the upper non-target tissue. By way of example, external devices (e.g., contact cooling) can maintain the skin surface at a temperature below the treatment temperature, and in some preferable aspects maintain the skin surface temperature in a range from about 38°C to about 42°C, or about 40°C. In some aspects, a cooling surface through which the electromagnetic radiation is applied to the skin can be placed into contact with the surface of the patient's skin to continuously cool the upper layers of the skin during the modulation of the EMR. Alternatively, contact cooling can be modulated via on and off pulses in concert with the delivery of radiation. The pulses can be repeated for the duration of the treatment.

In certain aspects, for example, it has been found that a skin-contact surface of the radiation applicator can be maintained at a higher temperature than previously believed to provide sufficient cooling of the skin to maintain the skin surface temperature at a range of about 38°C to about 42°C, or about 40°C during the treatment duration, while nonetheless maintaining the ability of the treatment region at depth to be maintained at a temperature in a range of about 42°C to about 47°C. The temperature of the cooling surface can be maintained at a variety of temperatures in a range of about 20°C to about 35°C, in some aspects, however, the temperature of the cooling surface can maintained in a range of about 25-35°C, or about 20-25°C, or about 35°C. By way of example, cooling elements (e.g., that can be disposed within a housing connected to the applicator via an umbilical) can maintain the skin-contacting surface of the applicator in a range of 20-30°C or about 20-25°C during treatment with radiation of a wavelength of about 1210 nm and at about 35°C during treatment with radiation of a wavelength of about 1060 nm via the circulation of cooling fluid maintained at or adjusted to be at the appropriate temperature. The temperature of the cooling surface is selected, in part, as a function of the selectivity of the wavelength being employed in a treatment and/or the power level employed in a certain treatment and/or the on/off time of the treatment.

Exemplary treatment regimes for generating target regions in accordance with various aspects of the present teaching will be discussed below with reference to the schematics of FIGS. 4B and 4C. With reference first to FIG. 4A, however, the typical treatment regime of a known, commercially-available device marketed is shown. FIG. 4A provides a depiction of a non-invasive fat reduction treatment using a 1060nm laser coupled with surface cooling (with cooling water temperature set at 15°C). A device having such characteristics is commercially available under the tradename SculpSure^{®}, and is discussed above in connection with FIG. 4A.

In general, the treatments, methods, operation, parameters and other innovative features and design elements recited herein are generally applicable to non-invasive, semi-invasive, and invasive body contouring and/or tissue modification / treatment systems. In general, such systems can use energy in the form of electromagnetic radiation, heat, electricity, and the cycling thereof to achieve the heating and cooling phases described herein. Reference to SculpSure systems, components, handpiece, etc., is for the purposes of illustration and/or comparison to the innovations described herein. The disclosure is not limited to one body contouring and/or tissue modification / treatment system.

Still referring to FIG. 4A, based on a standard protocol (e.g., for treatment of abdominal tissue), a deep target treatment region 401 is generated in the subcutaneous fat tissue in the hypodermis extending from below the level of the D/H junction (e.g., about 6 mm below the skin surface) to a depth about 2-3 cm below the skin surface, which target treatment region is maintained in a treatment temperature range of 42-47°C by applying to the skin surface laser radiation having a central wavelength of about 1060 nm and a power density between about 0.9 to about 1.4 W/cm². Treatment thickness range from the top depth of treatment labeled DA1 402, which is the below the dermal/hypodermal junction ranging from about 0.6 cm to about 1.5 cm from the surface of the skin to the bottom depth of treatment DA2 404, which measures from about 1.5 cm to about 3.5 cm from the surface of the skin. The median treatment depth DA3 403 measures from about 1.2 cm to about 2.2 cm from the surface of the skin. The skin thickness ranges from about 1 mm to about 4 mm. Peak temperatures were achieved at the depth of about 10-15 mm from the surface of the skin.

The heating and cooling settings in the system of FIG. 4A generate a heating profile in subcutaneous fat where a superficial layer of tissue (including skin and approximately 1cm fat) is cooled below hyperthermia range (42-47°C) and therefore prevents superficial tissue being damaged during treatment.

The temperature of the cooling water applied to the skin surface via a contact cooling applicator is maintained at about 15°C, which was effective to cool the skin surface temperature to a temperature in a range of about 20-25 °C. An initial build phase of about 4 minutes was utilized, for a total treatment time of 25 minutes. During the sustain phase, EMR was applied for 20 seconds and then removed for 10 seconds.

With reference now to FIG. 4B, an innovative exemplary treatment regime is depicted in accordance with various aspects of the present teachings. A relatively superficial or shallow target region 411 applicable for treatment of relatively shallow areas of fat tissue (e.g., submental) and/or skin tightening of tissue areas (including submental tissue), extends from below the level of the D/H junction 205. The top depth of treatment region labeled DB 1 412 which is below the dermal/hypodermal junction, ranging from about 0.4 cm to about 1 cm from the surface of the skin to the bottom depth of treatment region labeled DB2 414, which ranges from about 1 cm to about 3 cm from the surface of the skin. The median treatment depth labeled DB3 413 measures from about 0.8 cm to about 1.8 cm from the skin surface. The skin thickness ranges from about 1 mm to about 4 mm. The treatment temperature range of 42-47°C of the treatment region can be maintained by applying to the skin surface laser radiation having a central wavelength of about 1060 nm and a power density between about 1 to about 2.5 W/cm². Peak temperatures are achieved at the depth of about 0.8 cm to about 1.8 cm, or about 1 cm from the skin surface.

With respect to the procedure depicted in FIG. 4B, the temperature of the cooling water provided to the skin surface via a contact cooling applicator can be maintained at about 35°C, which can be effective to cool the skin surface temperature to a temperature in a range of about 38-42°C, or about 40°C. The cooling temperature of the applicator is nearly double the temperature of that used in the procedure depicted in FIG. 4A. An initial build phase of about 1.5 minutes was provided, and a total treatment time of 25 minutes. During the sustain phase, EMR was applied in on-off cycles of 5 seconds-5 seconds. The cooling in FIG. 4B, from cooling element 207, is directed relative to the epidermis and dermis as shown by cooling region 410.

In contrast with the known treatment regime of FIG. 4A, the innovative regime depicted in FIG. 4B confined the target region to a substantially smaller volume, centered about a shallower median treatment depth. Such an innovative treatment regime was found to be particularly effective for the treatment of regions of relatively shallowly located fat, such as fat in the submental region, and/or for tightening the skin in all areas (including abdomen and submental regions).

With reference now to FIG. 4C, another exemplary treatment regime in accordance with various aspects of the present teachings is depicted. A relatively superficial or shallow target region 415 applicable for treatment of lax skin tissue by tightening skin tissue (all skin tissue areas including submental tissue) is adjacent the D/H junction 205. A relatively superficial target region is selected to treat a shallow region of skin tissue to that extends from the level adjacent the D/H junction 205 to a depth ranging from about .5 cm to about 1.5 cm from the skin surface.

Further, the treatment thickness ranges from the top depth of treatment labeled DC1 416, which is adjacent the dermal/hypodermal junction ranging from about 0 cm to about 0.5 cm from the surface of the skin to the bottom depth of treatment labeled DC2 418, which ranges from about 0.5 cm to about 1.5 cm from the surface of the skin. The median treatment depth labeled DC3 417 measures from about 0.3 cm to about 0.8 cm from the skin surface. The skin thickness ranges from about 1 mm to about 4 mm.

The target region can be maintained in a treatment temperature range of 42-47°C by applying to the skin surface laser radiation having a central wavelength of about 1210 nm and a power density between about 1 to about 2.5 W/cm². Peak temperatures are achieved at the depth of from about 3 mm to about 8 mm, or about 6 mm. The temperature of the cooling water can be maintained at about 20-25°C, which can be effective to cool the skin surface temperature to a temperature in a range of about 38-42°C, about 40°C.

Still referring to FIG. 4C, an initial build phase of about from about 0.5-1.5 minutes was provided, and a total treatment time of 25 minutes. During the sustain phase, EMR was applied in on-off cycles of 5 seconds-5 seconds. Relative to the treatment region 411 of FIG. 4B, the treatment region 415 of FIG. 4C demonstrates an even more confined treatment region and sharper temperature gradient that is directly adjacent the D/H junction 205. Such a treatment regime was found to be particularly effective for the treatment of skin in all areas (including abdomen and submental regions).

Additional heating capacity (e.g., a heater, a heat exchanger) is added to various device, system and method embodiments to enable the applicator contact surface to achieve the 35°C temperature associated with FIG. 4B. This is an advantage and is a specific technical design feature to overcome the problem of existing designs that provide applicator contact surface cooling at 15°C, but that are unable to provide applicator contact surface cooling in the range of from about 20 to about 35°C.

The following examples are provided for further elucidation of various aspects of the present teachings. The examples are only for illustrative purposes and are not intended to indicate necessarily the optimal ways of practicing the present teachings or the optimal results that may be obtained.

### Example 1

### STUDY FOR TIGHTENING OF THE SKIN USING TWO NON-INVASIVE DEVICES

A study was completed to compare two different non-invasive light based aesthetic devices (e.g., two different wavelengths diodes) for skin tightening. The primary objectives of the two aesthetic devices was to (1) increase skin thickness as measured via Ultrasound (US), (2) confirm collagen deposition and elastin fibers deposition by histology, (3) provide reproducible results with a safe profile and (4) achieve acceptable patient tolerance.

This approach to a tightening treatment was to provide a hyper-thermic treatment to skin and underlying tissue using two light based devices to raise tissue temperature. Our hypothesis is that the controlled rise in temperature would induce a controlled thermal injury to the dermis and/or the hypodermis. The applicants believe that the controlled injury will stimulate collagen and elastin generation during the body's process of repairing the tissue previously damaged.

In this treatment study the two proposed devices were used to heat in vivo tissue to create a controlled injury. Long term tissue response post injury was studied to understand the safety and efficacy of such a treatment.

### DEVICE SPECIFICATIONS

| Device | Wavelength | Expected Irradiance | Mechanism |
|---|---|---|---|
| Device 1 | 1210 nm | 1-2.5 W/cm² | Maintain a moderate temperature rise for minutes in the region about the D/H junction |
| Device 2 | 1060nm | 1-2.5W/cm² | Maintain a moderate temperature rise for minutes in the region about D/H junction |

### Evaluation Methods

Several evaluation methods were employed in association with the study each evaluation method having its own purpose.

### Ultrasound (US)

A high frequency ultrasound imaging system was used to non-invasively measure skin changes. Ultrasound measurements were taken at predetermined treatment visits and the results were compared with the subjects' earlier results and with the results of the group of subject's overall. Applicants' observed hyperechoic patterns (e.g., cloudy portion of image indicative of inflammation) and encircled the region of the hyperechoic patterns in several of the US scanned images.

### Histology

H&E stain and elastin stain were used to evaluate tissue response post treatment (12 weeks post treatment). A pathologist reviewed the histology to evaluate changes in skin and hypodermis.

### Temperature monitoring

A thermal camera was used to measure temperature of the skin surface during treatment and immediately after treatment.

### SUBJECT POPULATION AND SELECTION CRITERIA

The study population included subjects presenting with loose abdominal skin who met inclusion criteria including, is a healthy male or female between 18 and 85 years old who is willing to undergo laser treatment for tissue tightening and have an abdominoplasty performed at the end of the study. The subjects consented to a surgical abdominoplasty procedure and all treatments and US tests were conducted on a human abdomen in vivo prior to the subjects scheduled abdominoplasty.

The subjects were treated three months before their scheduled abdominoplasty. Four areas of each subjects' abdomen that were to be treated were tattooed in in the respective treatment sample regions. Specifically, four permanent small tattoo marks the size of a pencil tip (about 2 mm) were placed at the edges of each of the proposed treatment area(s)) surrounding the navel. A baseline Ultrasound (US) image was taken of the area to be treated.

### First Treatment

Three months before abdominoplasty surgery the abdomen area was treated with the two devices (Device 1 and Device 2). Prior to and after irradiation with the Devices (Device 1 and Device 2) temperature monitoring of the skin surface was conducted with a thermal camera. At 6 weeks follow up, US imaging was taken to evaluate and to measure changes in the tissue. At 12 weeks follow up, US imaging was taken to evaluate and measure changes in the tissue. The patient underwent the aforementioned abdominoplasty. The patients' excised abdominal tissue previously treated in the tattooed treatment sample regions with Device 1 and Device 2 was sampled for histology analysis by a pathologist.

Each subject had a portion of their abdominal area treated with each of the two devices in the treatment sample regions. Treatment sample regions of the different devices did not overlap. The hand pieces were placed in contact with the skin. The hand piece settings are detailed in association with FIGS. 4B (Device 2) and 4C (Device 1) were used by the applicant to treat the subjects.

At the start of the study and after each treatment, standard post treatment instructions were reviewed with the subjects, these include: maintain your current weight and do not change your diet or exercise routine, aquaphor may be applied to the treatment area, clean area daily with mild soap and water and pat dry, do not rub or scratch the treatment area, and any discomfort may be relieved by using ice packs or acetaminophen.

### US Results within 1 week of treatment

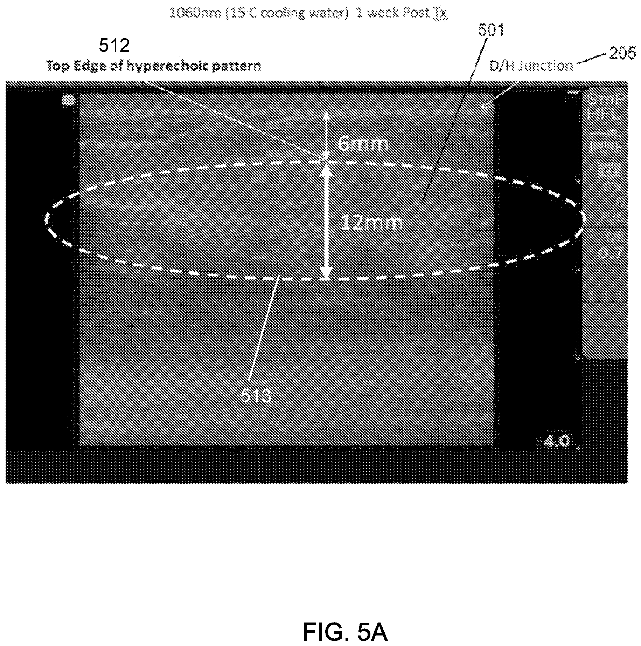

FIG. 5A shows an image of an abdominal area one week after it was treated with a device discussed with respect to FIG. 4A. The image shows that the inflammation 501 indicative of treatment with the device's laser (1060 nm, cooling water applicator temperature of 15°C, cooled skin surface temperature of 20-25°C, laser power density of 0.9-1.4 W/cm²) is at a depth of from the skin surface.

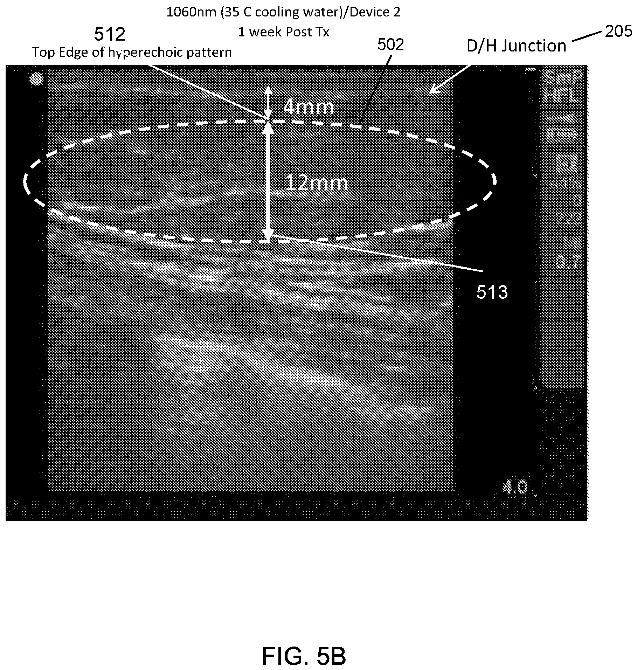

FIG. 5B shows an US image of an abdominal area treated with Device 2 discussed with respect to FIG. 4B (1060 nm, cooling water applicator temperature of 35°C, cooled skin surface temperature of 38-42°C, laser power density of 1-2.5 W/cm²) one week after a treatment. FIG. 5B shows that one week after treatment with Device 2 an encircled hyperechoic pattern in the US image (also called a cloudy image) indicative of strong tissue inflammation is at a distance away from the dermal/hypodermal junction. Specifically, the top edge of the encircled hyperechoic pattern measures at about 4 mm from the D/H junction 205. The distanced between the top edge and the bottom edge 513 of the encircled hyperechoic pattern measures about 12mm.

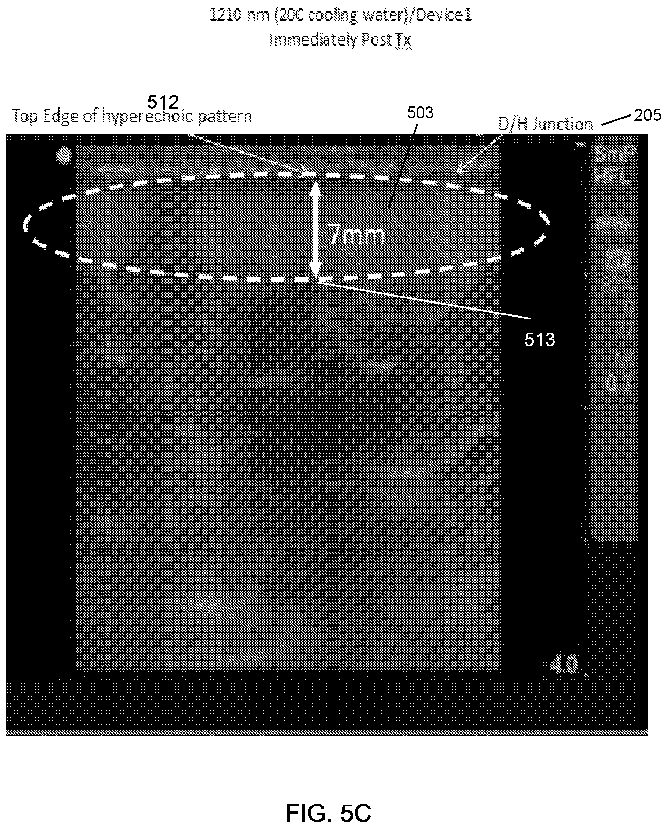

FIG. 5C shows an US image of an abdominal area treated with Device 1 and discussed with respect to FIG. 4C (1210 nm, cooling water applicator temperature of 20C, cooled skin surface temperature of 38-42C, laser power density of 1-2.5 W/cm2) immediately after treatment. FIG. 5C shows that immediately after treatment with Device 1 a hyperechoic pattern in the US image is indicative of a strong tissue inflammation that is located adjacent to the dermal/hypodermal junction. The encircled hyperechoic pattern in FIG. 5C shows that the top edge of the hyperechoic pattern is at the D/H junction. Thus, treatment of the abdominal area with Device 1 provides tissue inflammation results adjacent to the dermal/hypodermal junction in contrast to the US images of the treatment Device 2 shown in FIG. 5B, which show a hyperechoic pattern that is at a distance of about 4 mm below the D/H junction.

Further, the treatment thickness range shown in FIG. 5C with Device 1, which is measured from the top edge of the encircled hyperechoic pattern 503 to the bottom edge of the hyperechoic pattern 503 measures about 7 mm and this is more narrowly tailored than the hyperechoic pattern 502 treatment thickness range that is shown in FIG. 5B with Device 2, which measures 12 mm. Applicants believe that there is an advantage to having a narrowly tailored region of tissue inflammation as was found to result from a treatment with Device 1. The narrowly tailored treatment region avoids nodule formation and provides more intense heating in the smaller treatment region. Since the treatment is adjacent the D/H junction 205 and is narrowly tailored it is suited to tissue areas with shallow fat, because undesirable heating of organs, fascia, and/or other structures can be avoided.

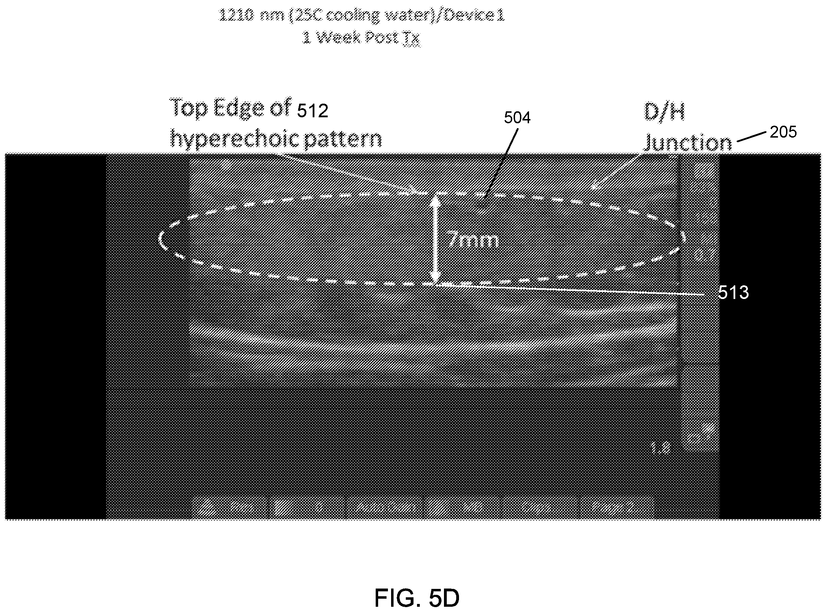

While the results of the treatments shown in FIGS. 5B and 5C were taken one week apart with the FIG. 5B results being one week post treatment with Device 2 and FIG. 5C showing results immediately after treatment with Device 1, the results of treatment with Device 1 immediately after treatment and one week after treatment have been shown to be substantially the same. Specifically, FIG. 5D shows US image results of a separate Device 1 treatment study then is shown in FIGS. 5A-5C where the US images of Device 1 as discussed with respect to FIG. 4C (1210 nm, cooling water applicator temperature of 25°C, cooled skin surface temperature of 38-42°C, laser power density of 1-2.5 W/cm2) were taken 1 week after treatment and these results are substantially the same as the results of treatment with Device 1 shown in FIG. 5C where the results were taken immediately after treatment.

Further, the results shown in FIG. 5D scanned 1 week after treatment also show that treatment with Device 1 provides tissue inflammation 504 results adjacent to the dermal/hypodermal junction and the treatment thickness range, which is measured from the top edge of the encircled hyperechoic pattern to the bottom edge of the hyperechoic pattern 504 similarly measures about 7 mm. Thus, the comparison of Device 1 and Device 2 results immediately after treatment and a week after treatment as shown in FIGS. 5B and 5C are appropriately compared.

### US AND HISTOLOGY RESULTS WITH DEVICE 1- 1210nm and cooled skin surface 38-42C

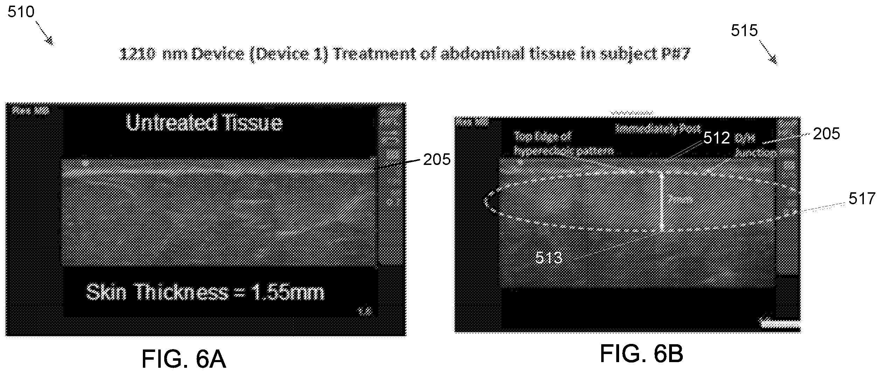

FIGS. 6A-6D show images of a single region of abdominal tissue in a single study subject having patient identification number P#7. The four corners of the tissue sample region were tattooed with dot sized markers to identify the area of treatment and to enable evaluation of this single region of tissue before and after treatment.

FIG. 6A shows the image 510 from scans of the untreated tissue 610 (e.g., baseline tissue) and using the scanned scan image the tissue thickness was measured at 1.55 mm thick. FIG. 6B shows the scan image 515 of the same tissue region immediately after treatment with the 1210nm device for substantially uniform heating of tissue (i.e., Device 1). FIG. 6B shows that immediately after treatment a hyperechoic pattern 517 in the US image (also called a cloudy image) indicative of strong tissue inflammation directly adjacent to the dermal/hypodermal junction is visible with the top edge of the hyperechoic pattern measuring at the depth of the dermal/hypodermal junction and the bottom edge of the hyperechoic pattern measuring a distance of 7 mm from the top edge. The bottom edge of the hyperechoic pattern indicative of tissue inflammation is at the depth of approximately 1cm deep from the skin surface.

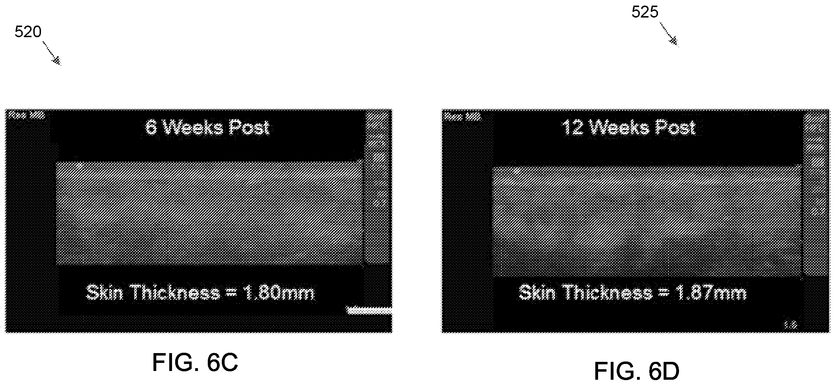

FIG. 6C shows the scan image 520 of the single region of tissue six weeks after the single treatment with Device 1, which indicates that the skin thickness measurement has increased to 1.80 mm thick from 1.5 mm thick in FIG. 6A and that the tissue has healed from the inflammation 601 observed immediately after treatment in FIG. 6B. The scan also indicates that there is continued healing of the tissue adjacent the dermal/hypodermal junction. Applicants believe that the continued presence of the hyperechoic pattern indicates that there has been continued healing of the controlled thermal injury provided by Device 1.

FIG. 6D shows the scan image 525 of the single region of tissue twelve weeks after the single treatment with Device 1, which indicates that the skin thickness measurement has continued to increase to measure 1.87 mm thick. The twelve week US scan images also show that the tissue has additionally healed adjacent to the dermal/hypodermal junction subsequent to the US scans previously conducted at six weeks.

Table 1 shows the change in skin thickness of a single skin region in three study subjects (having ID numbers P#7, P#8 and P#10) after a single treatment of a single region of abdominal tissue in a single study subject with Device 1 (as discussed in association with FIG. 4C) at 1210 nm with 20C cooling water.

### Skin Thickness (treatment with Device 1, 1210 nm)

**TABLE 1**

| ID | Baseline/mm | 6 weeks/mm | 12 weeks/mm |
|---|---|---|---|
| P#7 | 1.55 | 1.80 (16%) | 1.87 (21%) |
| P#8 | 1.43 | 1.64 (15%) | 1.66 (16%) |
| P#10 | 1.44 | 1.83 (27% | 1.68 (17%) |

The four corners of an abdominal tissue region were tattooed with dot sized markers to identify the treatment sample area and to enable evaluation of the treatment sample area (a single region of tissue) before and after treatment. US scans of the untreated tissue (e.g., baseline tissue) were taken and baseline thickness of the treatment area was measured. The data is shown as a column of Table 1 entitled Baseline/mm. The treatment area was treated with Device 1 and then US scans of the treatment area were taken six weeks after treatment and skin thickness measurements at six weeks after treatment were determined by the US scans and this data is shown as a column of Table 1 entitled 6 weeks/mm. After six additional weeks, a total of 12 weeks after the treatment of treatment area with Device 1, US scans of the treatment area were taken and skin thickness measurements at 12 weeks after treatment were determined by via US and this data is shown as a column of Table 1 entitled 12 weeks/mm.

Overall there is an increase in thickness at 12 weeks that is above 15%, ranging from 21%, 16% and 17% across patients P#7, P#8, and P#10 respectively. While patients P#7 and P#8 consistently showed an increase in thickness from baseline, to 6 weeks post treatment, to 12 weeks post treatment, patient P#10 shown a significantly greater increase from baseline at 6 week to 27% and a subsequent reduction from the thickness observed at 6 weeks to the thickness of 17% observed at 12 weeks. However, patient P#10 experienced a 17% increase from baseline at 12 weeks, which falls in the range of thickness observed via US in the other two discussed samples, P#7 and P#8 at 12 weeks from baseline after treatment with Device 1. Without being bound to any single theory, applicants submit that patient P#10 was likely experiencing edema as part of their bodies' specific healing response at 6 weeks post treatment.

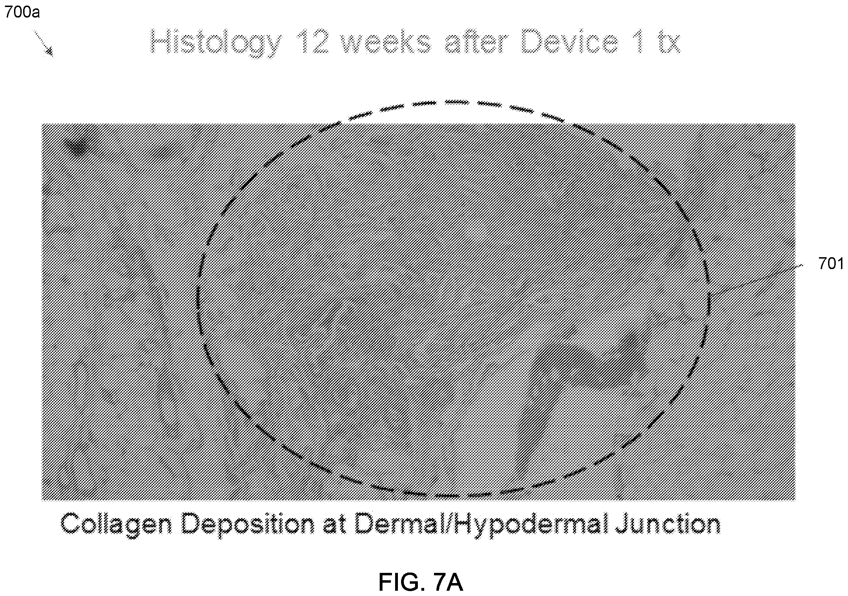

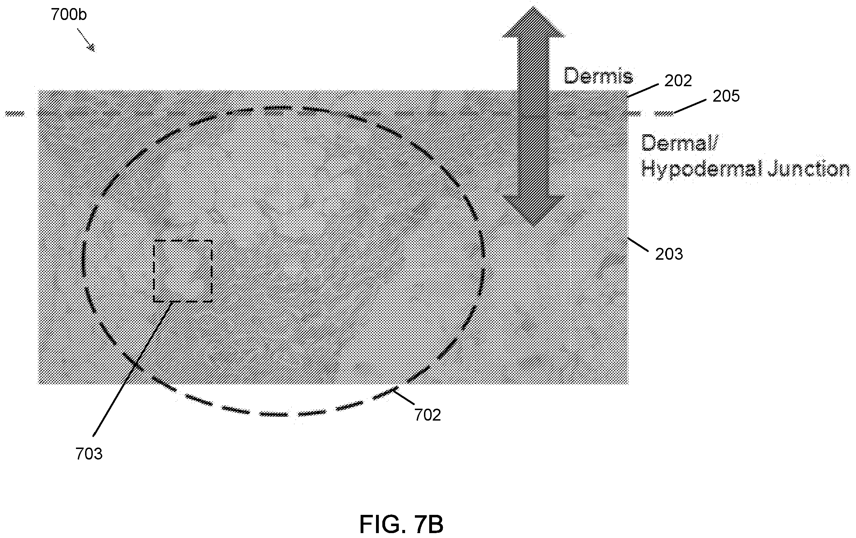

FIGS. 7A and 7B provide histology of the injury 700a 700b created by Device 1 (at 1210 nm with a uniform bream treatment protocol discussed in association with FIG. 4C). The histology was evaluated twelve weeks after a treatment. The histology shows collagen deposition 701, 702 directly adjacent to the dermal/hypodermal junction and highlights collagen deposition in an encircled region of the image. Fig. 7B depicts the location of the dermis and also depicts the D/H junction as a dotted line that falls below the dermis. Further, the histology image encircles the newly deposited collagen tissue 702 that is directly adjacent to the D/H junction. In FIG. 7A another portion of collagen deposition 703 adjacent to the dermal/hypodermal junction is encircled. The histology at the dermal/hypodermal junction show dense structures 703 that are indicative of the tissue repairing via new collagen deposition.

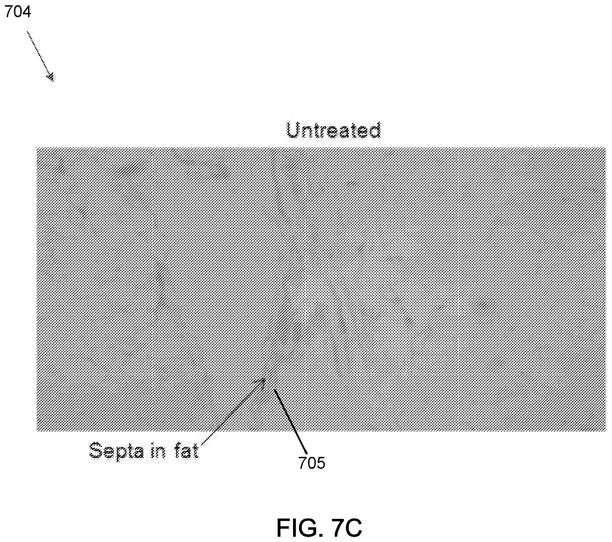

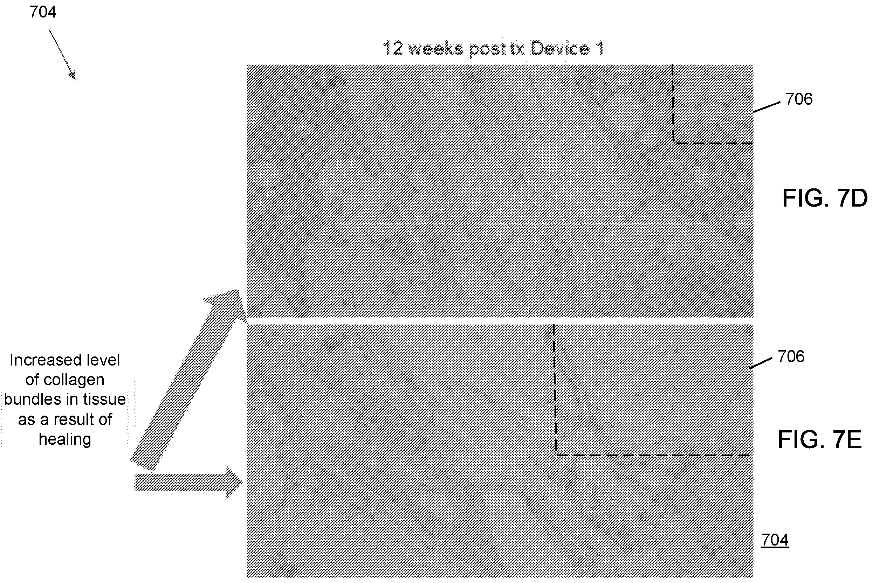

FIG. 7C provides histology of untreated abdominal tissue 704 that includes a region of septa 705 in the fat tissue. FIGS. 7D and 7E provide histology that is nearby the region of abdominal tissue 704 shown in FIG. 7C twelve weeks after the tissue has been treated with Device 1 (at 1210 nm and at 20°C cooling water and as described in association with FIG. 4C) in a single treatment. The histology shown in FIGS. 7D and 7E reveal an increased size of collagen bundles 706 in the abdominal tissue from the untreated sample of FIG. 7C after the treatment with Device 1 and also after the passage of twelve weeks of healing the treated area. The increase of collagen bundles 706 in FIGS. 7D and 7E provide clear histological support that the healing response to treatment of abdominal tissue 704 with Device 1 includes a remarkable increase in the bundles of collagen 706 present in the abdominal tissue 704 histology twelve weeks after treatment.

### US AND HISTOLOGY RESULTS WITH DEVICE 2- 1060nm and cooled skin surface 38-42C

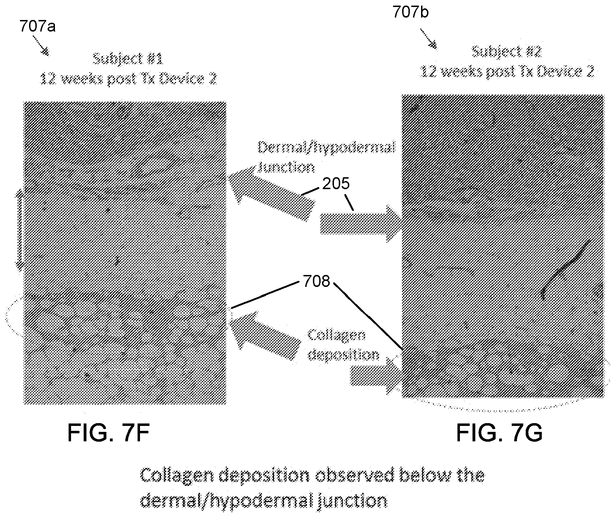

Table 2 and FIGS. 7F and 7G provide results from a separate study that also used Device 2 (as discussed in association with FIG. 4B). Table 2 provides a summary of skin thickness results at 12 weeks after a single treatment with Device 2 and FIGS. 7F and 7G provide histology data. While this is from a separate study than the current protocol, table 2 and FIGS. 7F and 7G are used to help show the relationship between skin thickness changes at 12 weeks after treatment with Device 2 and the histological changes after treatment with Device 2.

Table 2 shows results from 10 subjects that were treated on the abdomen with 1060nm laser and at 35°C water cooling temperature. The skin thickness was measured with US at baseline and 12 weeks. The data provided in Table 2 shows a large range in outcome from a 2% loss in thickness to a 20% gain in thickness, and an average gain of 7% in thickness.

### Skin Thickness

### Percentage change 12 weeks post Treatment(Tx) with 1060 nm at 35° C water cooling temperature (Device 2) as compared to baseline

**TABLE 2**

| Subject ID # | % Change |
|---|---|
| 15 | -1% |
| 19 | -1% |
| 23 | 11% |
| 32 | 7% |
| 33 | 10% |
| 16 | -2% |
| 20 | 4% |
| 22 | 20% |
| 27 | 18% |
| 35 | 7% |
| Average | 7% |

FIGS. 7F and 7G provides histology of the injury 707 created by Device 2 (at 1060 nm and 35°C with a uniform beam treatment protocol) of two additional subjects', subjects #1 and #2, treated abdominal tissue. The histology was evaluated twelve weeks after a treatment. The histology shows collagen deposition 708 at a distance of from about 2 mm to about 4mm below the dermal/hypodermal junction. The newly deposited collagen is encircled. The histology shows dense structures that are indicative of the tissue repairing via new collagen deposition. The histology of abdominal tissue treated with Device 2 shows that the injury is below and is not directly adjacent to the dermal/hypodermal junction.

The data show that treatment with Device 2 provides an average increase in skin thickness (see e.g., Table 2) and an increase in collagen deposition 708 as shown in FIGS 7F and 7G and applicants believe that increased skin thickness and increased collagen deposition will improve the appearance of the surface of the skin tissue from baseline. The collagen deposition provided by treatment with Device 2 occurs at a distance of from about 2 mm to about 4 mm below the D/H junction 205. Applicants also believe that improved thickness and collagen deposition indicative of healing is preferably directly adjacent to the dermal/hypodermal junction. This is because applicants believe that a more natural and smoother skin surface appearance will result from thickening and/or collagen deposition centered directly adjacent to the dermal/hypodermal junction. As a result, treatment with Device 1 provides a more natural and smoother skin appearance after treatment than treatment with Device 2, however, both Devices 1 and 2 provide an improved smoothed skin appearance over baseline.

In one embodiment, one or more combination treatments may be conducted on a single treatment region of a patient. In accordance with such combination treatments, the patient is first treated with one device and then after a healing period, for example, from about 6 six weeks, from about 12 weeks, from about 18 weeks, the patient is then treated with a different device. Suitable combinations may be used to treat the entire depth of tissue from the hypodermal tissue adjacent the D/H junction 205 down to a depth of about 30 mm from the skin surface.

For example, first a subject's tissue region A is treated with a SculpSure^{™} system or a another system suitable for performing the steps recited herein and twelve weeks after the treatment the subject is treated with a device similar to Device 1 described in association with Example 1 herein. In this way, the depth of a subject's treatment may be from the D/H junction 205 to a depth as deep as 35 mm from the skin surface. Therefore the subject can achieve both fat reduction and skin tightening effects in the tissue region A.

In another example, a second subject's tissue region B is treated with a device similar to Device 2 described in association with Example 1 herein and twelve weeks after the initial Device 2 treatment the patient is then treated with Device 1. In this way, the depth of a subject's treatment may be from the D/H junction 205 to a depth as deep as 30 mm from the skin surface. Therefore the subject can achieve both fat reduction and skin tightening effects in the tissue region B.

### NON-INVASIVE FAT REDUCTION IN THE SUBMENTAL AREA

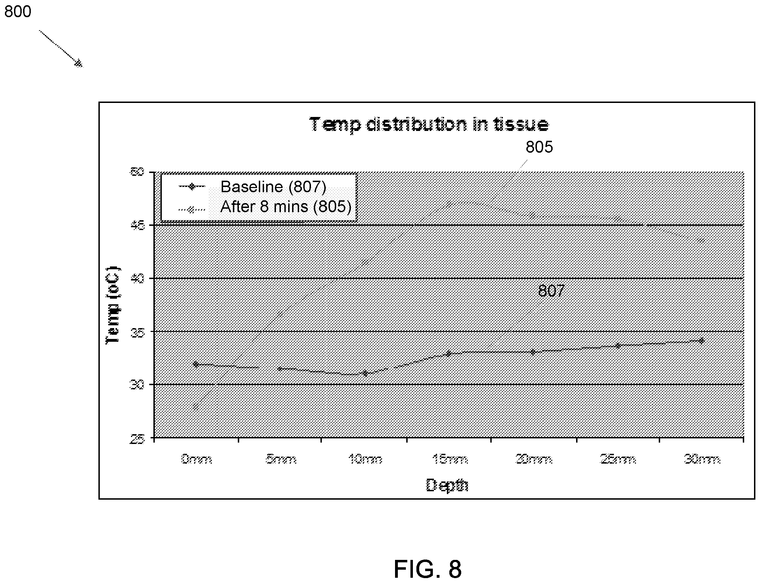

All of this is an admission. FIG. 8 demonstrates the spatial temperature distribution 800 at various depths in a subject's abdominal tissue after a 1060 nm treatment with surface cooling set at 15°C (i.e., a commercially available FDA cleared SculpSure treatment). The patient was under general anesthesia and a single thermocouple needle was employed to penetrate into the subject's abdominal tissue in the treatment region at different depths prior to and after treatment with the SculpSure product. The data show that the hyperthermia range is achieved at a depth ranging from about 11 mm to greater than 30mm from the skin surface. This design works well for treating large areas (such as abdomen, flanks etc.) where large amount of fat is present at a depth. However, for areas with only small pockets of fat, and/or a shallow pocket of fat tissue, such as the submental area, the current system / device heat/cooling settings and capabilities that are described in association with FIG. 4A, will not generate sufficient heat in the superficial layer of fat (e.g., in areas of shallow fat pockets) and therefore is limited in fat reduction efficacy for shallow areas. In addition, various commercially available body contouring systems are designed for deep tissue heating. These systems are unsuitable for targeting the tissue depths described herein. For example, such systems designed for deep tissue heating are not suitable for treating submental region, shallow superficial fat areas, and other similar areas and depths.

A new treatment design is needed to treat areas with thin layer of fat (approximately 1cm or less in fat thickness) and/or thin layers of fat that are concentrated in a relatively superficial and/or shallow depth. In order to concentrate more heat to superficial layer underneath the skin new treatment settings have been developed for use with various body contouring and tissue treatment systems. The cooling water temperature has been increased from a temperature of 15°C to up to 35°C. The laser heating settings including power and laser on/off schedule was also altered to raise the temperature in the superficial layer to achieve the hyperthermia range (42-47°C), and also to make the treatment tolerable to the subject. The conditions employed to achieve this goal of hyperthermic treatment of the superficial fat layer are described in association with FIG. 4B and were previously discussed in Example 1 in association with the trials conducted with Device 2.

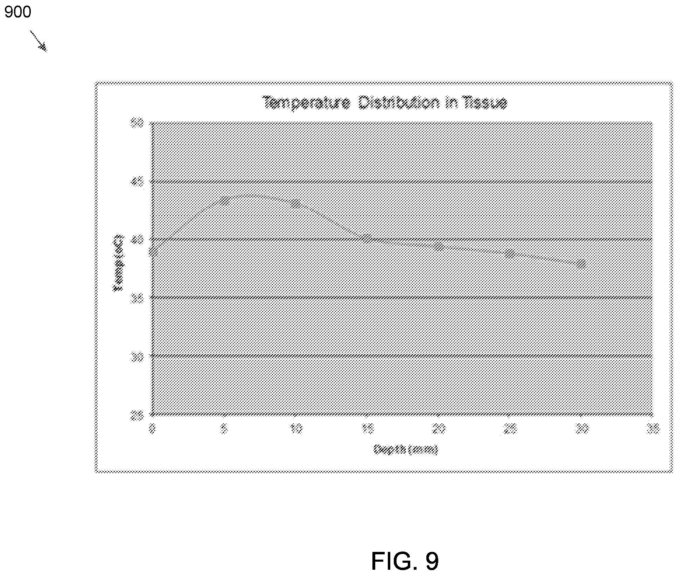

In order to observe its temperature profile, the device described in association with FIG. 4B (1060 nm and a 35°C applicator surface cooling temperature) was used to treat a subject's abdomen. FIG. 9 shows the temperature profile/temperature distribution 900 at various depths on a subject's abdomen after a treatment with a 1060 nm wavelength at a cooling surface temperature of 35°C. Specifically, examples of temperature distribution at various depths on a subject's abdomen post a 1060 nm laser radiation session are shown. With regard to the laser radiation session shown, surface cooling was set at 35oC. The patient was under general anesthesia and a single thermocouple needle was employed to penetrate into the subject's abdominal tissue in the treatment region at different depths prior to and after treatment with the 1060 nm wavelength device having a cooling surface of 35°C and described in association with FIG. 4B.

Hyperthermic treatment temperature range (42-47°C) is located at a depth ranging from about 2 mm to about 13mm from the skin surface. The relatively superficial depth of treatment achieved by the conditions associated with the device described in association with FIG. 4B makes this device suited for relatively shallow areas of fat, such as the submental area. Additional heating capacity (e.g., a heater, a heat exchanger) may be added to a given product, system or method embodiment facilitate the applicator contact surface achieving the 35°C temperature depicted in the temperature profile provided at FIG. 9.

### Example 2

Four Subjects having PATIENT ID Nos. 01, 03, 06 and 09 were treated on the submental area with a Device 3, a 1060nm laser coupled with surface cooling having a cooling temperature at 35°C (providing a skin surface temperature ranging from about 38-42°C, or about 40°C), having a power density ranging from 1.2-2.3W/cm², having a build phase of 1.5 minutes (laser on/off schedule: 25 seconds on /5 seconds off), having a sustain phase: 23.5 minutes (laser on/off schedule: 5 seconds on /5 seconds off), and having a total treatment time lasting 25 minutes. US and MRI imaging were used to evaluate the presence of injury to the fatty region of tissue and to demonstrate the location of the fat tissue (e.g., subcutaneous tissue, hypodermal tissue) injury post treatment. The treatment efficacy is evaluated using US measurement of fat thickness at baseline and 12 weeks post and visually using photographs of patients at baseline and at 12 weeks post treatment. Note, that the Device 3 used in Example 2 is similar to the Device 2 discussed in Example 1.

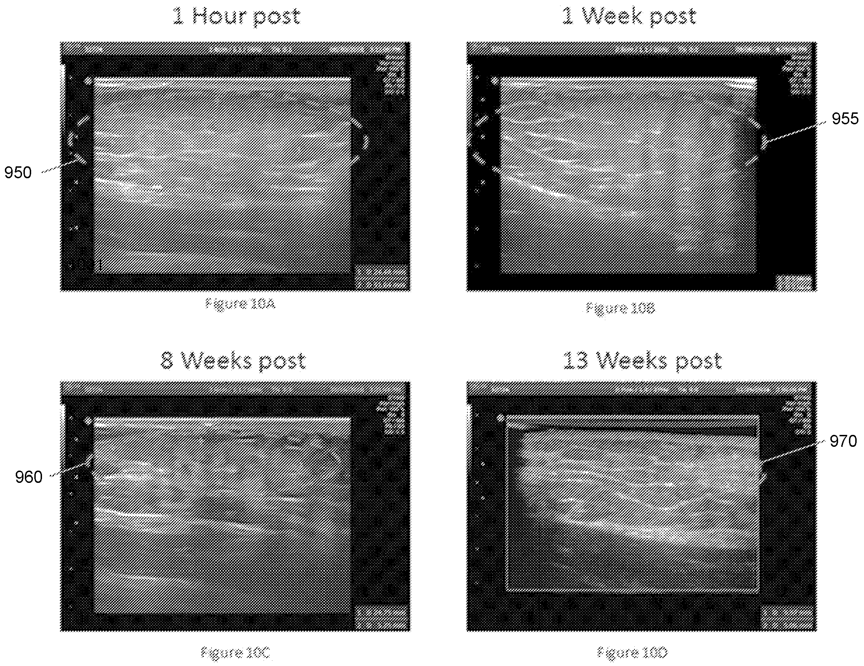

FIGS. 10A-10D illustrate a single patient, Patient ID No. 3, treated in the submental area with Device 3 at various time periods after treatment and as evaluated via US. FIG. 10A shows that 1 hour after treatment of the submental area with Device 3 a hyperechoic pattern 950, 955, 960,and 970, encircled in the figure, is visible at a depth below the D/H junction.

Likewise, FIG. 10B shows the same area 955 in the same patient 1 week after treatment of the submental area with Device 3 and the encircled hyperechoic pattern 955 is largely unchanged from that shown 1 hour after treatment in Figure 10A. The encircled hyperechoic pattern (e.g., the US cloud) shows that the Device 3 provides a relatively superficial or shallow tissue treatment.

FIG. 10C shows results after eight weeks following treatment of the submental area with Device 3 the encircled hyperechoic pattern 960 is becoming smaller, which is indicative of healing in the treated region.

FIG. 10D shows that 13 weeks after treatment of the submental area with Device 3 the encircled hyperechoic pattern 970 is becoming even smaller then was observed at 8 weeks after treatment in FIG. 10C and this indicates continued healing in the treated region.

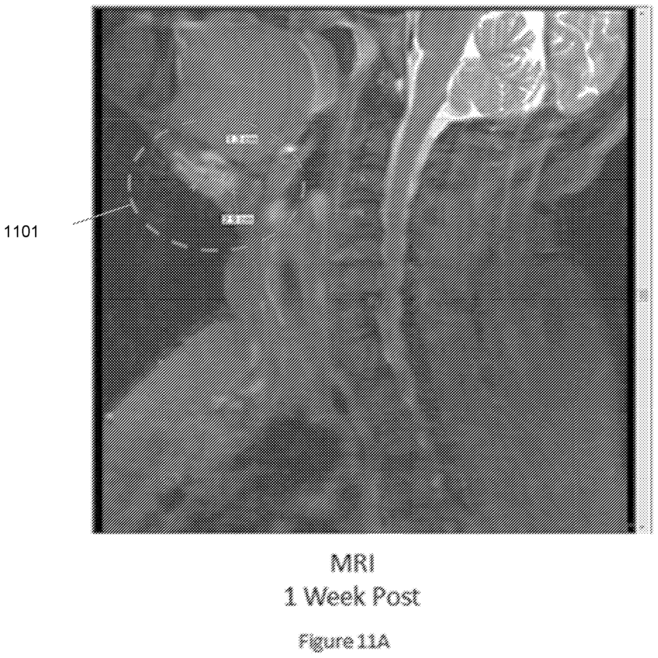

FIG. 11A shows that 1 week after treatment of the submental area with Device 3 an MRI scan of the treated submental area in Patient ID No. 03 shows that the encircled region 1101 of tissue is altered relative to untreated tissue. The encircled region 1101 provides an image that does not appear in natural untreated tissue, and applicants submit that this tissue change from natural tissue subsequent to treatment of the submental area with Device 3 is indicative of tissue inflammation.

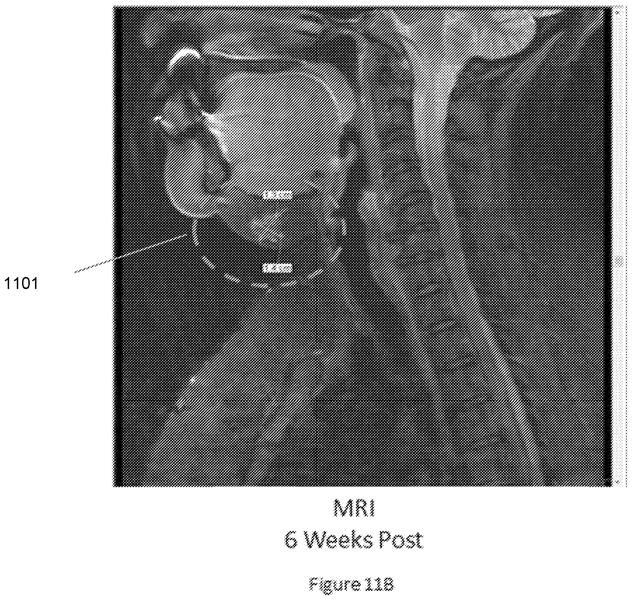

FIG. 11B shows that 6 weeks after treatment of the submental area with Device 3 an MRI scan of the treated submental area in Patient ID No. 03 shows that the encircled region 1101 of tissue is altered relative to untreated tissue. The encircled region provides an image that does not appear in natural untreated tissue, and applicants submit that this tissue change from natural tissue subsequent to treatment of the submental area with Device 3 is indicative of tissue inflammation and a healing response.

Table 3 provides a summary of US scan data measuring fat thickness in mm of four patients, Patient ID Nos. 01, 03, 06, and 09 at baseline prior to treatment with Device 3 and twelve weeks after treatment with Device 3. The results show a range of reduction in fat thickness from baseline at 12 weeks that ranges from 6%, 21%, 16%, and 20% reduction in fat thickness. The average fat thickness reduction was over a 15% reduction in fat thickness after a single treatment with Device 3 in the submental area.

### 12 Weeks US - 1060 nm and 35oC

**TABLE 3**

| Patient ID | Fat Thickness Baseline/mm | Fat Thickness 12 weeks post/mm | Changes |
|---|---|---|---|
| 01 | 10.30 | 9.68 | 6% |
| 03 | 8.15 | 6.42 | 21% |
| 06 | 10.43 | 8.75 | 16% |
| 09 | 6.40 | 5.10 | 20% |

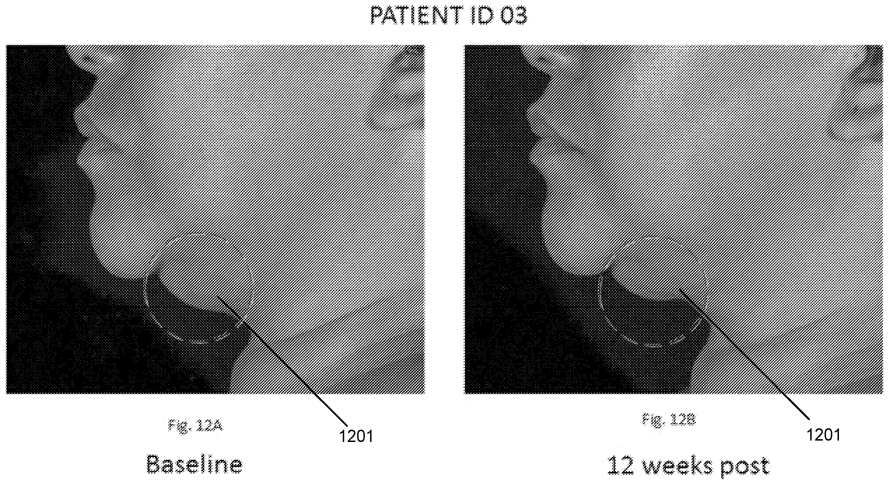

FIGS. 12A-12B are photographs of Patient ID No. 03 at baseline (FIG. 12A) and 12 weeks after a single treatment with Device 3 (FIG. 12B). The 21% reduction in fat in Patient ID No. 03 as determined via US and presented in Table 3 is visually observable as a noticeable reduction in bulge 1201 present in the subject's submental area. In addition, the reduction in fat has not created an undesired side effect of lax, loose or droopy skin in the treated submental area. To the contrary the skin in the region of the treated submental area appears to have "snapped back" and has tightened to conform to the treated area where the quantity of fat has been reduced.

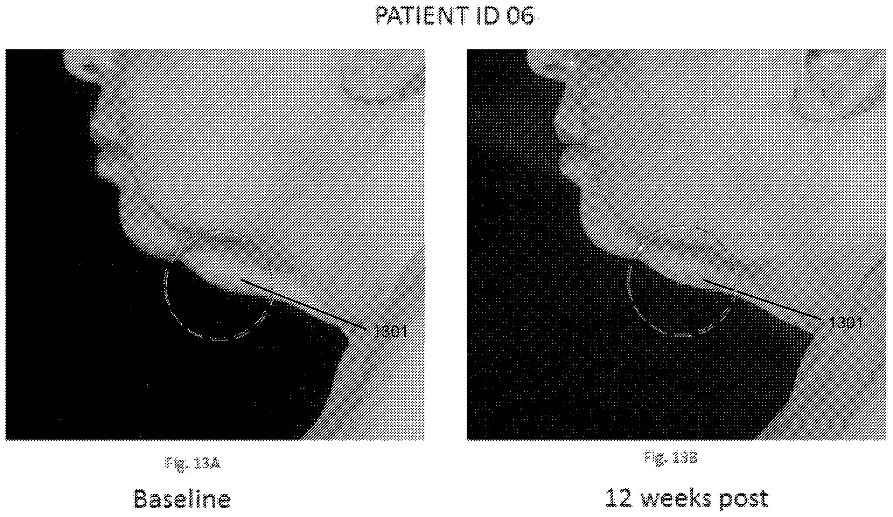

FIGS. 13A-13B are photographs of Patient ID No. 06 at baseline (FIG. 13A) and 12 weeks after a single treatment with Device 3 (FIG. 13B). The 16% reduction in fat in Patient ID No. 06 as determined via US and presented in Table 3 is visually observable as a noticeable reduction in bulge 1301 and present in the subject's submental area. In addition, the reduction in fat has not created an undesired side effect of lax, loose or droopy skin in the treated submental area. To the contrary the skin in the region of the treated submental area appears to have "snapped back" and has tightened to conform to the treated area where the quantity of fat has been reduced.

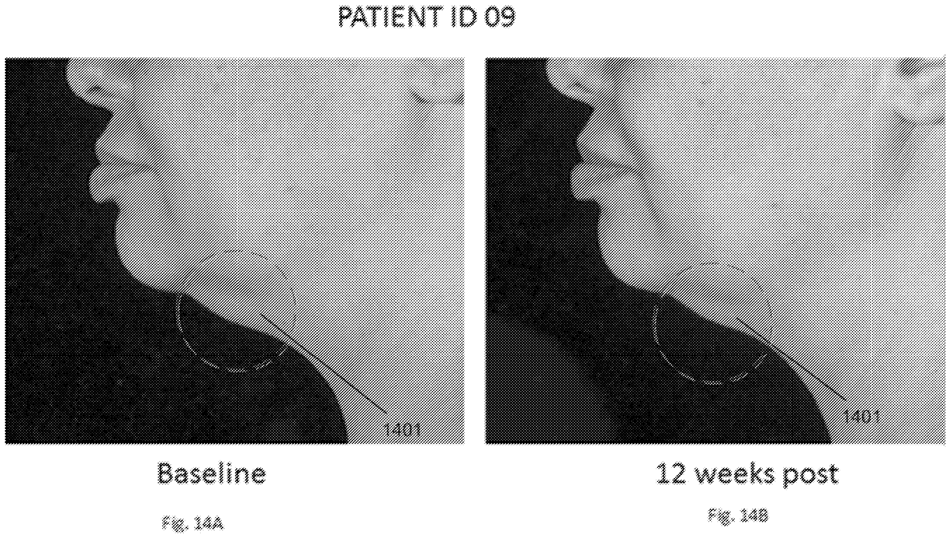

FIGS. 14A-14B are photographs of Patient ID No. 09 at baseline (FIG. 14A) and 12 weeks after a single treatment with Device 3 (FIG. 14B). The 20% reduction in fat in Patient ID No. 09 as determined via US and presented in Table 3 is visually observable as a noticeable reduction in bulge 1401 present in the subject's submental area. In addition, the reduction in fat has not created an undesired side effect of lax, loose or droopy skin in the treated submental area. To the contrary the skin in the region of the treated submental area appears to have "snapped back" and has tightened to conform to the treated area where the quantity of fat has been reduced.

Patients participating in the study of treatment of the submental area with Device 3 reported some short term swelling and skin warmth after treatment. Patients also reported tenderness in the treated area lasting couple of weeks. Finally, some subjects reported firmness in the treated area, but all reported firmness in the treated submental area resolved by 6 weeks after treatment.

In various aspects, because extended treatment times are utilized to perform the treatments described (e.g., about 25-30 minutes), various aspects of the present teachings provide systems and methods for a reliable, safe, and/or relatively comfortable photothermal treatment to the patient in a manner that is relatively hands-free and/or with relatively little oversight, thereby potentially reducing the costs associated with continued oversight by the practitioner. In addition, various aspects of the systems and methods disclosed enable customization so as to fit various body areas requiring treatment and/or the isolation of the target treatment area.

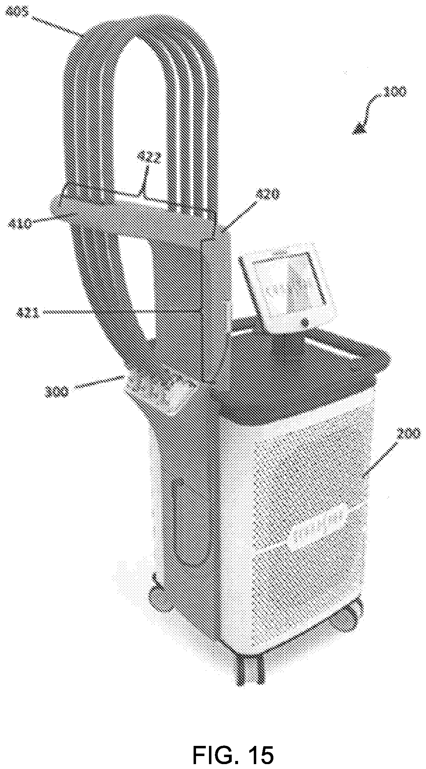

Referring now to FIG. 15, an exemplary system 100 for the non-invasive (or less-invasive) photothermal treatment for skin tightening and fat reduction is depicted. As shown system 100 represents certain aspects of a device or system as described, for example in U.S. Patent Pub. No. 20170266461 entitled "Systems and Methods of Unattended Treatment of a Subject's Head or Neck", and modified in accordance with various aspects of the present teachings is depicted. Additional exemplary approaches to photothermal treatment of tissue at depth and modified for use in accordance with methods and systems of the present teachings are disclosed, for example, in U.S. Pub. No. 20070213792 entitled "Treatment of Tissue Volume with Radiant Energy"; U.S. Pub No. 20080103565 entitled "Method and Apparatus for Treatment of Cutaneous and Subcutaneous Conditions"; U.S. Patent Pub. No. 20140025033 entitled "Non-Invasive Fat Reduction by Hyperthermic Treatment"; U.S. Patent No. 7,276,058 entitled "Method and Apparatus of Treatment of Cutaneous and Subcutaneous Conditions" issued on October 2, 2007; U.S. Patent No. 7,351,252 entitled "Method and Apparatus for Photothermal Treatment of Tissue at Depth" issued on April 1, 2008; and U.S.

Patent No. 8,915,948 entitled "Method and Apparatus for Photothermal Treatment of Tissue at Depth" issued on December 23 2014, the teachings of which may be used in the context of the invention.

As shown in FIG. 15, the exemplary system 100 for the non-invasive treatment of undesired body fat generally includes a housing 200 that can contain one or more sources of electromagnetic radiation (not shown), a plurality of umbilical cords 405 extending therefrom, and one or more applicators 300 coupled to the distal end of the umbilical cords 405 for applying the treatment radiation to the patient's skin when disposed in contact with the surface of the treatment region. Though the depicted exemplary system includes four applicators, any of a number of applicators 300 can be included in the system, for example, one applicator, two applicators, four applicators, or more. When not in use, the plurality of applicators 300 can be stored in a dock on the housing 200. Suitable energy sources can be, for example, temperature control (e.g., cooling and/or heating), light based energy sources, electromagnetic radiation, RF energy, and ultrasound energy, as known in the art and modified in accordance with the present teachings. As discussed in detail below, the treatment energy generated by the EMR source(s) can be delivered to the applicator, for example, via an optical waveguide (e.g., optical fiber) coupled to the EMR source(s) and extending through the umbilical cord 405.

As shown in FIG. 15, the system 100 additionally comprises an arm 420 extending from the housing 200 that can support at least a portion of the umbilical cords 405, for example, above the subject to be treated and/or at a desired distance from the patient and/or other portions of the system including, for example, the housing 200 containing the energy source. It will be appreciated that umbilical cords 405 for use in accordance with the present teachings can have a variety of configurations but generally define a conduit through and are sufficiently flexible such that they can be maneuvered into a desired position.

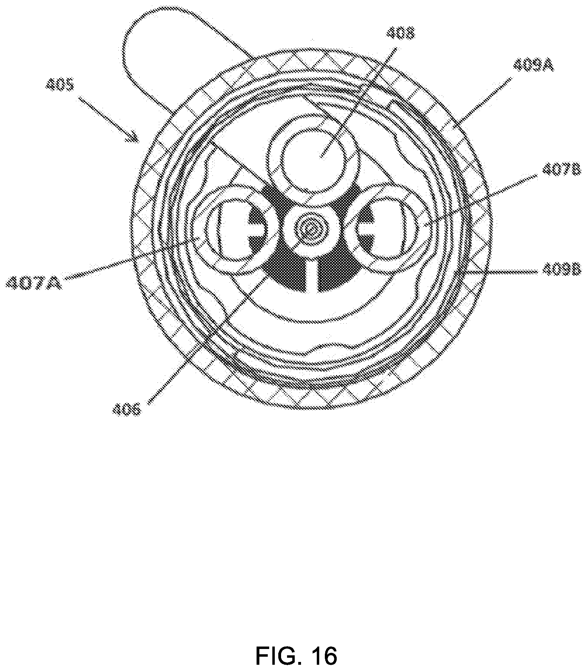

By way of example, as shown in FIG. 16, the exemplary umbilical cord 405 comprises a corrugated, flexible outer surface 409A (e.g., made of plastic) as well as a corrugated, inner shell 409B that is also flexible but can be made of a material (e.g., metal, stainless steel) that provides increased protection to the fibers and/or conduit extending through the conduit defined by the umbilical cord 405. For example, FIG. 16 depicts that an optical waveguide (e.g., optical 406) extends through the conduit for delivering EMR from the EMR sources to the applicators. Additionally, as discussed in detail below, one or more fluid pathways 407A-B can extend through the conduit, for example, for delivering cooling fluid to and returning cooling fluid from the applicator 300. Additionally, one or more signal cables 408 can be provided to enable electric communication between the housing 200 and the applicator 300 (e.g., including for transmitting signals generated by contact sensors of the applicators).

As discussed above, it may also be desirable to cool the skin-contacting surface 307 of the applicator 300 so as to cool the layers of the skin above the target region at depth. In some aspects, for example, as discussed above with reference to FIG. 16, one or more fluid pathways 407A-B can extend through the conduit, for example, for delivering cooling fluid to the applicator 300 for maintaining the skin-contacting surface and/or the skin surface at a desired temperature (e.g., to confine the hyperthermic treatment to the target tissue while keeping temperatures of dermal tissue above the targeted tissue at depth below injury threshold). In such aspects, the housing 200 of FIG. 15 can include a heater and/or chiller for maintaining the cooling fluid at a desired temperature.

Further, by way of example, a temperature regulating heater/chiller can maintain the cooling fluid in a range of about 15°C to about 35°C depending on the desired treatment. In some aspects, the temperature of the cooling surface is maintained in a range of about 25°C to about 30°C. Alternatively, the temperature of the cooling surface can be maintained at a temperature of about 35°C. By way of example, cooling elements (e.g., that can be disposed within a housing connected to the applicator via an umbilical) can maintain the skin-contacting surface of the applicator in a range of 25-30°C during treatment with radiation of a wavelength of about 1210 nm and at about 35°C during treatment with radiation of a wavelength of about 1060 nm via the circulation of cooling fluid maintained at or adjusted to be at the appropriate temperature.

Additionally, where the applicator surface is cooled, the use of contact sensors can prevent unwanted heating (e.g., in the epidermal and/or dermal layer) due to lack of contact and/or incomplete contact between the skin surface and the cooled applicator surface. Suitable approaches to cooling the skin during photothermal treatment and modified for use in accordance with methods and systems of the present teachings are disclosed, for example, in U.S. Patent No. 6,517,532 entitled "Light Energy Delivery Head" issued on February 11, 2003; U.S. Patent No. 6,663,620 entitled "Light Energy Deliver Head" issued on December 16, 2003; U.S. Patent No. 6,653,618 entitled "Contact Detecting Method and Apparatus for an Optical Radiation Handpiece" issued November 25, 2003; U.S. Patent No. 6,974,451 entitled "Light Energy Delivery Head" issued on December 13, 2005; U.S. Patent No. 6,976,985 entitled "Light Energy Delivery Head" issued on December 30, 2005; U.S. Patent No. 7,351,252 entitled "Method and Apparatus for Photothermal Treatment of Tissue at Depth" issued on April 1, 2008; U.S. Patent No. 7,763,016 entitled "Light Energy Delivery Head" issued on July 27, 2010; U.S. Patent No. 8,002,768 entitled "Light Energy Delivery Head" issued on August 23, 2011; U.S. Patent No. 8,915,948 entitled "Method and Apparatus for Photothermal Treatment of Tissue at Depth" issued on December 23 2014; U.S. Pub No. 20080103565 entitled "Method and Apparatus for Treatment of Cutaneous and Subcutaneous Conditions"; U.S. Pub. No. 20070213792 entitled "Treatment of Tissue Volume with Radiant Energy"; and U.S. Pub. No. 20140025033 entitled "Non-Invasive Fat Reduction by Hyperthermic Treatment," the teachings of which may be used in the context of the invention.

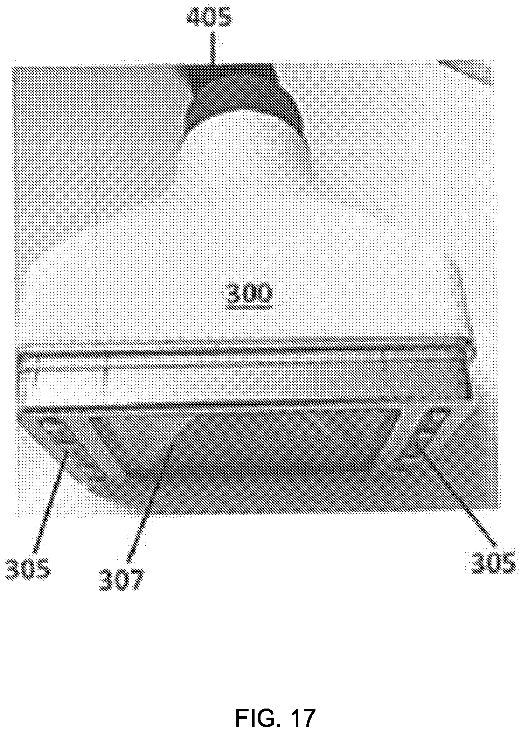

Referring now to FIG. 17, the exemplary applicator 300 of FIG. 17 is depicted in additional detail. As shown in FIG. 17, the applicator 300 (or treatment head) is coupled to the umbilical cord 405 (e.g., for delivery of the treatment energy) and includes an optical window having a skin-contacting surface 307 through which the treatment energy is transmitted from the applicator 300 to the treatment region. The optical window can have a variety of configurations but generally comprises a material

(e.g., glass, sapphire) selected to provide good optical coupling with the skin when in contact therewith. It will also be appreciated that the contact surface 307 of the applicator 300 can have a variety of sizes and shapes (e.g., depending on the surface to be treated) including rectangular, square, triangular, circular, oval, ellipse, trapezoid, rhombus, pentagon, hexagon, octagon, or parallelogram, all by way of non-limiting example. As shown in FIG. 17, for example, the contact surface is rectangular, and can have a short side that ranges from about 1 cm to about 10 cm and a long side that range from about 2 cm to about 15 cm.

It will be appreciated in view of the present teachings that the exemplary systems and methods disclosed herein can include one or more additional features to facilitate treatment of a patient as otherwise discussed above. Exemplary features include contact sensors and removable coupling for ease of application and/or unattended treatment. In light of the particularly efficacious treatment on a variety of body areas as provided herein, systems and methods can be customized or configured to treat specific treatment regions (e.g., abdomen, torso, flanks, below the bra area, arms, legs, or portions of the face, chin, and neck area including the submental area, the jowls, and chin). Additional details regarding these and other features of the exemplary system 100 depicted in FIG. 15 can be found in U.S. App. No. 15/485,178 (filed on April 11, 2017).

Although the preceding and following text sets forth a detailed description of different embodiments of the disclosure, it should be understood that the legal scope of the invention is defined by the words of the claims set forth at the end of this patent. The detailed description is to be construed as exemplary only and does not describe every possible embodiment of the disclosure since describing every possible embodiment would be impractical, if not impossible. Numerous alternative embodiments could be implemented, using either current technology or technology developed after the filing date of this patent, which would still fall within the scope of the claims defining the invention.

The aspects, embodiments, features, and examples of the disclosure are to be considered illustrative in all respects and are not intended to limit the disclosure, the scope of which is defined only by the claims. Other embodiments, modifications, and usages will be apparent to those skilled in the art without departing from the scope of the claimed invention.

The use of headings and sections in the application is not meant to limit the disclosure; each section can apply to any aspect, embodiment, or feature of the disclosure.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components and can be selected from a group consisting of two or more of the recited elements or components. Further, it should be understood that elements and/or features of a composition, an apparatus, or a method described herein can be combined in a variety of ways without departing from the scope of the present teachings, whether explicit or implicit herein.

The use of the terms "include," "includes," "including," "have," "has," or "having" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise.

It should be understood that the order of steps or order for performing certain actions is immaterial so long as the present teachings remain operable. Moreover, two or more steps or actions may be conducted simultaneously.

Where a range or list of values is provided, each intervening value between the upper and lower limits of that range or list of values is individually contemplated and is encompassed within the disclosure as if each value were specifically enumerated herein. In addition, smaller ranges between and including the upper and lower limits of a given range are contemplated and encompassed within the disclosure. The listing of exemplary values or ranges is not a disclaimer of other values or ranges between and including the upper and lower limits of a given range.

While the foregoing figures and examples refer to specific elements, this is intended to be by way of example and illustration only and not by way of limitation. It should be appreciated by the person skilled in the art that various changes can be made in form and details to the disclosed embodiments without departing from the scope of the teachings encompassed by the appended claims.

## Claims

1. A non-invasive body contouring system, comprising:
a housing (200);
a source of electromagnetic radiation configured to generate treatment energy and, disposed within the housing an applicator (300);
a cooling surface for contacting skin and being suitable for passing EMR therethrough;
an umbilical cord (405) extending from the housing (200) and coupled at a distal end to the applicator;
the applicator (300) being configured to contact a surface of a body and deliver treatment energy thereto, wherein the umbilical cord (405) is configured to receive treatment energy from the source and deliver the treatment energy to the applicator,
wherein the applicator (300) is configured to initially apply electromagnetic radiation to a skin surface for a duration sufficient to initially raise a temperature of a target region at or below a dermis/hypodermis junction to a hyperthermic temperature in a range from about 42 °C to about 47 °C;
wherein the applicator (300) is further configured to thereafter maintain the target region within the hyperthermic temperature range for a treatment duration from about 20 minutes to about 30 minutes, wherein the applicator is configured to maintain the temperature of the target region within the hyperthermic temperature range by modulating the electromagnetic radiation applied to the skin surface so as to cyclically cool and heat the target region, the treatment comprising a cooling phase and a heating phase,
wherein the system is configured to provide electromagnetic radiation to be employed during the heating phase a) in a power density range of 1 W/cm² to 5 W/cm², or b) in a power density range of 0.5 W/cm² to 10 W/cm² and in the wavelength range of 800 to 1300 nm;
wherein the duration of the cooling phase is in a range from about 3 seconds to about 15 seconds and the duration of the heating phase is in a range from about 3 seconds to about 15 seconds; and
wherein the applicator (300) is configured to terminate application of electromagnetic radiation to the skin after the treatment duration.

2. The system of claim 1, configured to treat the target region comprising the dermis/hypodermis junction at a depth in a range of about 3 mm to about 1 cm below the skin surface.

3. The system of claim 1, configured to treat the target region comprising fat tissue below the dermis/hypodermis junction at a depth in a range of about 1 cm to about 3 cm below the skin surface.

4. The system of claim 1, wherein the system is configured to modulate the electromagnetic radiation applied to the skin surface by adjusting the power density of the electromagnetic radiation applied to the skin surface between a first power density for a cooling duration and a second power density for a heating duration so as to cyclically cool and heat the target region.

5. The system of claim 4, wherein the second power density of the electromagnetic radiation is in a range between about 1 W/cm² and about 2 W/cm².

6. The system of claim 5, wherein the first power of the electromagnetic radiation is substantially zero.

7. The system of claim 1, wherein the system is configured to maintain temperature of the cooling surface in a range of about 15 °C to about 35 °C.

8. The system of claim 7, configured to maintain the temperature of the cooling surface in a range of about 25 °C to about 30 °C.

9. The system of claim 1, wherein the system is configured to provide a treatment duration in a range from about 20 minutes to about 25 minutes.

10. The system of claim 1, wherein the system is configured to provide the duration of the cooling phase in a range from about 3 seconds to about 10 seconds and the duration of the heating phase in a range from about 3 seconds to about 10 seconds.

11. The system of claim 1, wherein the system is configured to provide the duration of the cooling phase:
(a) in a range from about 3 seconds to about 7 seconds and the duration of the heating phase in a range from about 3 seconds to about 7 seconds; or
(b) in a range from about 3 seconds to about 6 seconds and the duration of the heating phase in a range from about 3 seconds to about 6 seconds; or
(c) about 5 seconds and the duration of the heating phase about 5 seconds; or
(d) about 4 seconds and the duration of the heating phase about 6 seconds.

12. The system of claim 1, wherein the electromagnetic radiation exhibits at least one wavelength in the near infrared range, optionally wherein the electromagnetic radiation exhibits a wavelength:
(a) of about 1210 nm; or
(b) selected from the group consisting of 800 nm, 940 nm, and 1060 nm.

13. The system of claim 1, wherein the system is configured to apply the electromagnetic energy to the skin surface to initially raise the temperature of the target region to the hyperthermic temperature for a duration in a range of about 20 seconds to about 40 seconds.

14. The system of claim 1, wherein the applicator (300) is a handpiece.

15. The system of claim 1, wherein one or more components of the applicator (300) is configured to deliver electromagnetic radiation and absorb heat.

## Patentansprüche

1. Nicht-invasives Körperformungssystem, umfassend:
ein Gehäuse (200);
eine Quelle elektromagnetischer Strahlung, die zur Erzeugung von Behandlungsenergie ausgelegt ist, und einen Applikator (300), der in dem Gehäuse angeordnet ist;
eine Kühlfläche zum Berühren von Haut und geeignet, um elektromagnetische Strahlung durchzulassen;
ein Nabelkabel (405), das sich von dem Gehäuse (200) erstreckt und an einem distalen Ende mit dem Applikator verbunden ist;
wobei der Applikator (300) zur Berührung einer Oberfläche eines Körpers und zur Bereitstellung von Behandlungsenergie an diese ausgelegt ist, wobei das Nabelkabel (405) zur Aufnahme von Behandlungsenergie von der Quelle und zur Lieferung der Behandlungsenergie an den Applikator ausgelegt ist,
wobei der Applikator (300) zur anfänglichen Anlegung elektromagnetischer Strahlung an eine Hautoberfläche für eine Zeitdauer ausgelegt ist, die ausreicht, um eine Temperatur eines Zielbereichs an oder unterhalb eines Haut-/Unterhaut-Übergangsbereichs auf eine hyperthermische Temperatur in einem Bereich von ungefähr 42°C bis ungefähr 47°C zu erhöhen;
wobei der Applikator (300) weiter ausgelegt ist, um den Zielbereich danach in dem hyperthermischen Temperaturbereich für eine Behandlungsdauer von ungefähr 20 Minuten bis ungefähr 30 Minuten zu halten, wobei der Applikator ausgelegt ist, die Temperatur des Zielbereichs in dem hyperthermischen Temperaturbereich zu halten, indem er die an die Hautoberfläche angelegte elektromagnetische Strahlung moduliert, so dass der Zielbereich periodisch gekühlt und erwärmt wird, wobei die Behandlung eine Kühlphase und eine Erwärmungsphase umfasst,
wobei das System ausgelegt ist, um anzulegende elektromagnetische Strahlung während der Erwärmungsphase a) in einem Leistungsdichtebereich von 1 W/cm² bis 5 W/cm², oder b) in einem Leistungsdichtebereich von 0.5 W/cm² bis 10 W/cm² und im Wellenlängenbereich von 800 bis 1300 nm bereitzustellen;
wobei die Dauer der Kühlphase in einem Bereich von ungefähr 3 Sekunden bis ungefähr 15 Sekunden liegt und die Dauer der Erwärmungsphase in einem Bereich von ungefähr 3 Sekunden bis ungefähr 15 Sekunden liegt; und
wobei der Applikator (300) zur Beendigung der Anlegung elektromagnetischer Strahlung an die Haut nach der Behandlungsdauer ausgelegt ist.

2. System nach Anspruch 1, das zur Behandlung des den Haut-/Unterhaut-Übergangsbereich umfassenden Zielbereichs in einer Tiefe in einem Bereich von ungefähr 3 mm bis ungefähr 1 cm unter der Hautoberfläche ausgelegt ist.

3. System nach Anspruch 1, das zur Behandlung des Zielbereichs, der Fettgewebe unterhalb des Haut-/Unterhaut-Übergangsbereichs umfasst, in einer Tiefe in einem Bereich von ungefähr 1 cm bis ungefähr 3 cm unter der Hautoberfläche ausgelegt ist.

4. System nach Anspruch 1, wobei das System zur Modulation der an die Hautoberfläche angelegten elektromagnetischen Strahlung ausgelegt ist, indem die Leistungsdichte der an die Hautoberfläche angelegten elektromagnetischen Strahlung zwischen einer ersten Leistungsdichte für eine Kühldauer und einer zweiten Leistungsdichte für eine Erwärmungsdauer angepasst wird, so dass der Zielbereich periodisch gekühlt und erwärmt wird.

5. System nach Anspruch 4, wobei die zweite Leistungsdichte der elektromagnetischen Strahlung in einem Bereich zwischen ungefähr 1 W/cm² und ungefähr 2 W/cm² liegt.

6. System nach Anspruch 5, wobei die erste Leistung der elektromagnetischen Strahlung im Wesentlichen null ist.

7. System nach Anspruch 1, das weiter eine Kühlfläche umfasst, wobei der Applikator (300) zur Anlegung der elektromagnetischen Strahlung durch diese Kühlfläche hindurch auf die Oberfläche der Haut des Patienten ausgelegt ist, sobald der Zielbereich in dem hyperthermischen Temperaturbereich gehalten wird, wobei das System zum Halten der Temperatur der Kühlfläche in einem Bereich von ungefähr 15°C bis ungefähr 35°C ausgelegt ist.

8. System nach Anspruch 7, das zum Halten der Temperatur der Kühlfläche in einem Bereich von ungefähr 25°C bis ungefähr 30°C ausgelegt ist.

9. System nach Anspruch 1, wobei das System zur Bereitstellung einer Behandlungsdauer in einem Bereich von ungefähr 20 Minuten bis ungefähr 25 Minuten ausgelegt ist.

10. System nach Anspruch 1, wobei das System zur Bereitstellung der Dauer der Kühlphase in einem Bereich von ungefähr 3 Sekunden bis ungefähr 10 Sekunden und der Dauer der Erwärmungsphase in einem Bereich von ungefähr 3 Sekunden bis ungefähr 10 Sekunden ausgelegt ist.

11. System nach Anspruch 1, wobei das System zur Bereitstellung der Dauer der Kühlphase ausgelegt ist:
(a) in einem Bereich von ungefähr 3 Sekunden bis ungefähr 7 Sekunden und der Dauer der Erwärmungsphase in einem Bereich von ungefähr 3 Sekunden bis ungefähr 7 Sekunden; oder
b) in einem Bereich von ungefähr 3 Sekunden bis ungefähr 6 Sekunden und der Dauer der Erwärmungsphase in einem Bereich von ungefähr 3 Sekunden bis ungefähr 6 Sekunden; oder
(c) ungefähr 5 Sekunden und der Dauer der Erwärmungsphase von ungefähr 5 Sekunden; oder
(d) ungefähr 4 Sekunden und der Dauer der Erwärmungsphase von ungefähr 6 Sekunden.

12. System nach Anspruch 1, wobei die elektromagnetische Strahlung zumindest eine Wellenlänge im nahen Infrarotbereich aufweist, wobei die elektromagnetische Strahlung optional eine Wellenlänge aufweist:
(a) von ungefähr 1210 nm; oder
(b) ausgewählt aus der Gruppe bestehend aus 800 nm, 940 nm und 1060 nm.

13. System nach Anspruch 1, wobei das System zur Anlegung der elektromagnetischen Energie an die Hautoberfläche ausgelegt ist, um die Temperatur des Zielbereichs zunächst auf die hyperthermische Temperatur für eine Dauer in einem Bereich von ungefähr 20 Sekunden bis ungefähr 40 Sekunden zu erhöhen.

14. System nach Anspruch 1, wobei der Applikator (300) ein Handstück ist.

15. System nach Anspruch 1, wobei ein oder mehrere Bauteile des Applikators (300) zur Lieferung von elektromagnetischer Strahlung und zur Aufnahme von Wärme ausgelegt ist/sind.

## Revendications

1. Système non invasif de remodelage corporel, comprenant :
un boîtier (200) ;
une source de rayonnement électromagnétique configurée pour générer de l'énergie de traitement et, disposé à l'intérieur du boîtier, un applicateur (300) ;
une surface de refroidissement destinée à entrer en contact avec la peau, et qui est apte à laisser passer le rayonnement électromagnétique, EMR, à travers celle-ci ;
un cordon ombilical (405) s'étendant à partir du boîtier (200) et couplé au niveau d'une extrémité distale à l'applicateur ;
l'applicateur (300) étant configuré pour entrer en contact avec la surface d'un corps et y délivrer une énergie de traitement, dans lequel le cordon ombilical (405) est configuré pour recevoir l'énergie de traitement de la source et délivrer l'énergie de traitement à l'applicateur,
dans lequel l'applicateur (300) est configuré pour appliquer initialement un rayonnement électromagnétique à la surface de la peau pendant une durée suffisante pour augmenter initialement la température d'une région cible à la jonction derme/hypoderme, ou en dessous de celle-ci, à une température hyperthermique située dans une plage comprise entre environ 42°C et environ 47°C ;
dans lequel l'applicateur (300) est en outre configuré pour maintenir par la suite la région cible dans la plage de température hyperthermique pendant une durée de traitement comprise entre environ 20 minutes et environ 30 minutes, dans lequel l'applicateur est configuré pour maintenir la température de la région cible dans la plage de température hyperthermique en modulant le rayonnement électromagnétique appliqué à la surface de la peau de manière à refroidir et chauffer cycliquement la région cible, le traitement comprenant une phase de refroidissement et une phase de chauffage,
dans lequel le système est configuré pour fournir un rayonnement électromagnétique à employer pendant la phase de chauffage a) dans une plage de densité de puissance de 1 W/cm² à 5 W/cm², ou b) dans une plage de densité de puissance de 0,5 W/cm² à 10 W/cm² et dans la plage de longueur d'onde de 800 à 1300 nm ;
dans lequel la durée de la phase de refroidissement se situe dans une plage comprise entre environ 3 secondes et environ 15 secondes et la durée de la phase de chauffage se situe dans une plage comprise entre environ 3 secondes et environ 15 secondes ; et
dans lequel l'applicateur (300) est configuré pour mettre fin à l'application du rayonnement électromagnétique à la peau après la durée de traitement.

2. Système de la revendication 1, configuré pour traiter la région cible comprenant la jonction derme/hypoderme à une profondeur située dans une plage comprise entre environ 3 mm et environ 1 cm sous la surface de la peau.

3. Système de la revendication 1, configuré pour traiter la région cible comprenant le tissu adipeux sous la jonction derme/hypoderme à une profondeur située dans une plage comprise entre environ 1 cm et environ 3 cm sous la surface de la peau.

4. Système de la revendication 1, dans lequel le système est configuré pour moduler le rayonnement électromagnétique appliqué à la surface de la peau en ajustant la densité de puissance du rayonnement électromagnétique appliqué à la surface de la peau entre une première densité de puissance pour une durée de refroidissement et une seconde densité de puissance pour une durée de chauffage, de manière à refroidir et chauffer cycliquement la région cible.

5. Système de la revendication 4, dans lequel la seconde densité de puissance du rayonnement électromagnétique se situe dans une plage comprise entre environ 1 W/cm² et environ 2 W/cm².

6. Système selon la revendication 5, dans lequel la première densité de puissance du rayonnement électromagnétique est sensiblement nulle.

7. Système de la revendication 1, dans lequel le système est configuré pour maintenir la température de la surface de refroidissement dans une plage comprise entre environ 15°C et environ 35°C.

8. Système de la revendication 7, configuré pour maintenir la température de la surface de refroidissement dans une plage comprise entre environ 25°C et environ 30°C.

9. Système de la revendication 1, dans lequel le système est configuré pour fournir une durée de traitement dans une plage comprise entre environ 20 minutes et environ 25 minutes.

10. Système de la revendication 1, dans lequel le système est configuré pour fournir la durée de la phase de refroidissement dans une plage comprise entre environ 3 secondes et environ 10 secondes, et la durée de la phase de chauffage dans une plage comprise entre environ 3 secondes et environ 10 secondes.

11. Système de la revendication 1, dans lequel le système est configuré pour fournir la durée de la phase de refroidissement :
(a) dans une plage comprise entre environ 3 secondes et environ 7 secondes, et la durée de la phase de chauffage dans une plage comprise entre environ 3 secondes et environ 7 secondes ; ou
(b) dans une plage comprise entre environ 3 secondes et environ 6 secondes, et la durée de la phase de chauffage dans une plage comprise entre environ 3 secondes et 6 environ secondes ; ou
(c) pendant environ 5 secondes, et la durée de la phase de chauffage pendant environ 5 secondes ; ou
(d) pendant environ 4 secondes, et la durée de la phase de chauffage pendant environ 6 secondes.

12. Système de la revendication 1, dans lequel le rayonnement électromagnétique présente au moins une longueur d'onde dans la bande de l'infrarouge proche, éventuellement dans lequel le rayonnement électromagnétique présente une longueur d'onde :
(a) d'environ 1210 nm ; ou
(b) choisie dans le groupe constitué de 800 nm, 940 nm et 1060 nm.

13. Système de la revendication 1, dans lequel le système est configuré pour appliquer l'énergie électromagnétique à la surface de la peau afin d'augmenter initialement la température de la région cible jusqu'à la température hyperthermique pendant une durée qui se situe dans une plage comprise entre environ 20 secondes et environ 40 secondes.

14. Système de la revendication 1, dans lequel l'applicateur (300) est une pièce à main.

15. Système de la revendication 1, dans lequel un ou plusieurs composants de l'applicateur (300) sont configurés pour délivrer un rayonnement électromagnétique et absorber de la chaleur.
